# EUROPEAN PATENT APPLICATION

(11) **EP 3 530 291 A1**
(43) Date of publication of application: **28.08.2019**
(21) Application number: 18158789.0
(22) Date of filing: 27.02.2018
(51) Int. Cl.: A61K 49/00, A61K 47/60, A61K 47/59, A61K 47/69

(54) **BI-FUNCTIONALIZABLE AMPHIPHILIC DIBLOCK COPOLYMERS, CONJUGATES AND USES THEREOF**

(71) Applicant: UNIVERSITE DE GENEVE, 1211 Geneva 4 (CH)
(72) Inventor: THAUVIN, Cédric, 68000 Colmar (FR); MAUDENS, Pierre, 02250 Marle (FR); ALLÉMANN, Eric, 1256 Troinex (CH)
(74) Representative: reuteler & cie SA

(57) **Abstract**

The present invention is directed to new bi-functionalizable amphiphilic di-block copolymers, conjugates, preparations, compositions, kits and uses thereof. In particular, the invention relates to compositions able to form self-assemblies useful as delivery systems for at least one bioactive agent.

## Description

### Field of the Invention

The present invention relates to bi-functionalizable amphiphilic di-block copolymers, preparations, conjugates, compositions and uses thereof.

### Background of the Invention

Extensive development has been conducted in the field of biocompatible and biodegradable polymers. In particular, biodegradable polyesters including polylactic acid (PLA), poly(lactic-co-glycolic acid) (PLGA) and polycaprolactone (PCL) are implemented frequently in pharmaceutical development due to their bioresorbable and biocompatible characteristics, enabling extensive applications in many fields such as tissue engineering, for example in medical sutures, bone fixation materials, and drug delivery. They are respectively obtained either by polycondensation of lactic acid, lactic and glycolic acids, or 6-hydroxyhexanoic acid or by ring-opening polymerization (ROP) of lactide, lactide and glycolide, or ε-caprolactone. However, the applications of those polymers are somehow limited due to their weak hydrophilicity, long degradation time, and low loading ability for polar drugs.

Therefore, co-polymers comprising a hydrophobic biodegradable polyester polymer and a biocompatible hydrophilic polymer, such as polyethylene glycol (PEG) have been developed to reduce opsonization of PLA polymeric nanoparticles after their intravenous injection. Thereby, PEG was used as a stealth agent bound to the PLA polymeric nanoparticles, resulting in improved hydrophilicity, degradation rate, and crystallization as compared to the hydrophobic biodegradable polyester polymer alone, thereby increasing the potential use in drug delivery.

Block copolymers of PEG and polyesters have many interesting properties, such as amphiphilicity, biocompatibility, biodegradability, and self-assembly in an aqueous environment under different structures such as micelles, polymersomes, capsules, nano-containers or micro-containers in an aqueous environment according to the length of their blocks. In particular, the addition of a PEG moiety to PLA, PLGA or PCL (Xiao et al., 2010, Int. J. Nanomedicine, 5, 1057-1065, doi:10.2147/IJN.S14912*;* Zhang et al., 2014, Journal of Controlled Release, 183, 77-86, doi:10.10161/j.jconrel.2014.03.026*, Wang et al., 2017, doi: 10.1016*/*j.polymdegradstab.2017.04.015*) allowed the resulting amphiphilic diblock copolymers to form self-assemblies in aqueous media when the hydrophilic/lipophilic balance is respected (*Xiao et al., 2010, supra*). Those copolymers have various applications in the pharmaceutical field, in particular when used as building blocks of nanocarriers or microcarriers.

For further applications, it would be desirable to link those polymers to molecules of interest such as dyes, fluorophores, therapeutic or diagnosing agents and depending on the applications and the property of the therapeutic or diagnosing agents, it might be desirable to have those molecules exposed to the surfaces of the nanocarriers or buried inside of the nanocarrier formed by the amphiphilic copolymers or have one type of molecules exposed and at the same time another type of molecules to be buried inside of a nanocarrier. However, the currently available di-block copolymers (e.g., PLA-PEG, PLGA-PEG, and PCL-PEG) do not provide two functionalizable groups on both sides of the copolymers. The functionalization of the di-block co-polymers on both sides has several limitations such as protection/deprotection steps, low yield and degree of substitution. Moreover, to get two functional groups on the resulting nanocarriers requires the use of two different mono-functionalizable copolymers.

Therefore, there is a need of developing functionalizable amphipathic di-block copolymers allowing an easy and cost-effective covalent conjugation of various functional moieties of interest (fluorophores, drugs, targeting molecules, etc.) on each side of the amphiphilic copolymer, depending whether the functional moiety is to be encapsulated in the nanocarrier formed by the self-assembling of the copolymer or rather exposed at its surface.

### Summary of the invention

The present invention relates to the unexpected finding that it is possible to add functionalizable groups (e.g., azide, alkyne, maleimide, biotin) on both sides of amphiphilic di-block copolymers comprising a biodegradable polyester and a hydrophilic biocompatible polymer, while preserving the ability of the resulting bi-functionalizable amphiphilic di-block copolymers to form self-assemblies in aqueous media. More interestingly, this observation was still true for conjugates resulting from the covalent coupling of those bi-functionalizable amphiphilic di-block copolymers with different fluorescent dyes by copper-free click chemistry leading to bi-functionalized nano-self-assemblies. The bi-functionalizable amphiphilic di-block copolymers of the invention are easy to obtain in a cost-effective manner and represent a very advantageous tool for forming, in a simple way and one step, bi-functionalized nano-self-assemblies in aqueous media (e.g., micelles) for a broad range of applications such as medical applications (such as ocular, topical, parenteral applications, as well as intraoperative applications), cosmetic applications and potentially other fields.

An aspect of the invention provides a bi-functionalizable amphiphilic di-block copolymer according to the invention.

Another aspect of the invention relates to a conjugate comprising a bi-functionalizable amphiphilic di-block copolymer of the invention covalently conjugated with at least one bioactive active agent.

Another aspect of the invention relates to a pharmaceutical or cosmetic composition comprising at least one bi-functionalizable amphiphilic di-block copolymer or at least one conjugate of the invention and at least one pharmaceutically acceptable carrier, diluent or excipient thereof.

Another aspect of the invention relates to a use of a bi-functionalizable amphiphilic di-block copolymer of the invention for the preparation of a cosmetic composition or a pharmaceutical composition.

Another aspect of the invention relates to a pharmaceutical or cosmetic composition comprising at least one bi-functionalizable amphiphilic di-block copolymer or at least one conjugate of the invention for use as a medicament.

Another aspect of the invention relates to a pharmaceutical or cosmetic composition comprising at least one bi-functionalizable amphiphilic di-block copolymer or at least one conjugate of the invention for use in *in vivo* delivery of medical agents, the diagnosis or prevention and/or treatment of disease or medical disorder, *in vitro* cell culture and cell and tissue engineering applications.

Another aspect of the invention relates to a method of preparation of a bi-functionalizable amphiphilic di-block copolymer, a conjugate thereof, a composition thereof, micelles or nanoparticles thereof according to the invention.

Another aspect of the invention relates to a method of preparation of a conjugate comprising the step of coupling a bi-functionalizable amphiphilic di-block copolymer of the invention with a bioactive agent.

Another aspect of the invention relates to a method of delivering a bioactive agent in a medium or body fluid or tissue, said method comprising contacting a conjugate according to the invention with said medium or body fluid or tissue, wherein the bioactive agent is released in said medium when the conjugate of the invention is biodegraded.

Another aspect of the invention relates to a method of preventing, treating or ameliorating a medical disorder, said method comprising administering in a subject in need thereof an effective amount of at least one conjugate of the invention or a pharmaceutical formulation thereof.

Another aspect of the invention relates to a kit comprising at least one bi-functionalizable amphiphilic di-block copolymer or composition thereof, e.g., in a lyophilized form.

Another aspect of the invention relates to a kit for preparation of nano/micro-carriers for encapsulation of material, comprising at least one least one bi-functionalizable amphiphilic di-block copolymer or a composition thereof (e.g., in an aqueous medium).

### Description of the figures

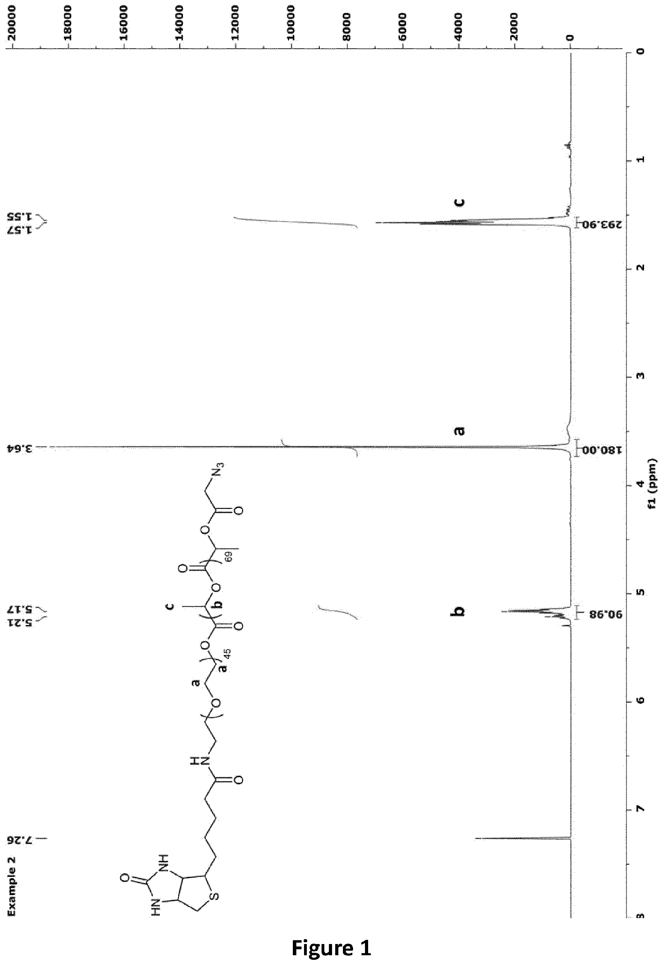

**Figure 1** shows ¹H NMR spectrum of polymer **(2)** described in Examples 1 and 2.

### Detailed description

The term "biodegradable polyester" refers to a biodegradable bio-based aliphatic polyester, including polylactic acid (PLA), poly lactic-co-glycolic acid (PLGA), poly-ε-caprolactone (PCL), polyhydroxybutyrate (PHB), and poly(3-hydroxy valerate) and derivatives thereof such as described in Rydz et al., 2015, Int J Mol Sci., 16(1), 564-596*,* in particular a polyester comprising optionally substituted lactide, optionally substituted glycolide or optionally substituted caprolactone monomers

The term "hydrophilic biocompatible polymer" encompasses hydrophilic biocompatible polymer based on polyethylene glycols (PEGs), poly(amino acid)s such as poly(glutamic acid) (PGA), poly(hydroxyethyl-L-asparagine) (PHEA) or poly(hydroxyethyl-L-glutamine) (PHEG), hyaluronic acid, chitosan, polysarcosine, poly(glycerol), poly(2-oxazoline)s and vinyl polymers such as poly(acrylamide), poly(vinylpyrrolidone) and poly(N-(2-hydroxypropyl)methacrylamide.

The term "polyethylene glycol (PEG)" refers to a polymer made of a chain comprising from 2 to about 12,000 ethylene oxide units, preferably 20 to 120 ethylene oxide units.

The term "hyaluronic acid" (HA) refers to linear heteropolysaccharide composed of D-glucuronic acid and D-N- acetylglucosamine, which are linked together *via* alternating β-1,4 and β-1,3 glycosidic bonds. Suitable HA polymers according to the invention encompass polysaccharides composed of up to 12,500 disaccharide repeats, and range in size from about 500 to 5,000,000 Da depending on the origin, in particular from about 500 to 3,000,000 Da. Each repeat unit bears a carboxylic chemical group on the D-glucuronic acid motif which can be potentially linked to form a graft polymer according to the invention. According to a particular aspect, the degree of substitution of those carboxylic groups with a linker of the invention is about 0.5-50 %, typically from 2 to about 10 %.

The term "chitosan" refers to a polymer made of a chain of D-glucosamine and N-acetyl-D-glucosamine with a total molecular weight ranging from 500 to about 327,000 Da, preferably from 500 to 5,000 Da.

The term "poly(amino acid)s" refers to a polymer made of a chain of amino acids units with a total molecular weight ranging from 500 to about 160,000 Da, preferably from 500 to 5,000 Da.

The term "polysarcosine" refers to a polymer made of a chain of sarcosine units with a total molecular weight ranging from 500 to about 160,000 Da, preferably from 500 to 5,000 Da. The term "poly(2-oxazoline)s" refers to a polymer made of a chain of 2-methyl-2-oxazoline or 2-ethyl-2-oxazoline or 2-propyl-2-oxazoline units with a total molecular weight ranging from 2,000 to about 500,000 Da, preferably from 2,000 to 10,000 Da.

The term "vinyl polymers" refers to a polymer made of a chain of vinyl monomers with a total molecular weight ranging from 500 to about 360,000 Da, preferably from 500 to 5,000 Da.

The term "degree of crosslink or substitution" as used herein means the quantity of pairs of functional groups converted into crosslinking or grafting bonds relative to the total quantity of pairs of functional groups initially present on the HA and the N-isopropylacrylamide based polymer, expressed as a percentage.

Suitable HA polymers according to the invention can be of various origins, including natural (generally animal), semi-synthetic or biotechnological (such as non-animal, produced by microorganism fermentation).

The term "functionalizable groups" refers to chemical groups or moieties that can be reacted, coupled or conjugated in aqueous medium to another group, for example through an addition reaction such as by click chemistry (copper free) or by interaction, to a reacting group of a bioactive agent for forming either a covalent coupling or a complex with the said reacting group. In a particular embodiment, the functionalizable groups are selected from alkynes such as C8 cycloalkynyls, C8 heterocycloalkynyls, azides, carboxylic acids, thiols, maleimide, biotin, avidin, halogen, amines, acrylates, active esters, epoxides, folic acid, hydrazides, nitrophenyl carbonate, ortho-pyridyl disulfide, protected amines (Fmoc, tBOC), vinyl sulfone, sulfonate (tosyl, mesyl), and silanes.

In particular, those groups include groups comprising an azide, alkyne, maleimide or a biotin moiety that can be subjected to a click addition (e.g., such as azide-alkyne addition).

The term "click chemistry" refers to a variety of chemical reactions (e.g., azide-alkyne cycloadditions) that are characterized by their high yield, regioselectivity, stability, and ability to proceed without the generation of offensive byproducts as described in Kolb et al., 2001, Chem. Int. Ed Engl., 40(11), 2004-2021*.* Importantly, these reactions can occur in a benign solvent, are insensitive to water and oxygen, and have a high thermodynamic driving. The most common examples of these reactions are the carbon-heteroatom bond formation resulting from cycloadditions of unsaturated species, nucleophilic substitution chemistry, non-aldol carbonyl chemistry, and additions to carbon-carbon multiple bonds. The conjugates of the invention can be synthesized by applying click chemistry to a bi-functionalizable amphiphilic di-block copolymer of the invention and examples of synthetic routes are provided herein, which can be slightly adapted by the skilled person using standard knowledge.

The term "conjugated" is used to indicate the formation of covalent bonds between the bi-functionalizable amphiphilic di-block copolymer of the invention and a bioactive agent leading to a conjugate of the invention, wherein such covalent bonding occurs through the functionalizable groups of the bi-functionalizable amphiphilic di-block copolymer of the invention.

The term "bioactive active agent" refers to a group or a molecule having a biological or medical application and may be selected from a macromolecular compound or a small molecule compound, such as peptides, proteins, oligo- and polynucleotides, lipids, antibiotics, antimicrobials, growth factors, enzymes, antitumoral drugs, anti-inflammatory drugs, antiviral drugs, antibacterial, antifungal drugs, anaesthetics, anti-neoplastic drugs, analgesics, anticoagulants, haemostatic drugs, dyes, fluorophores and a targeting molecule that recognizes a specific biomarker such as antibodies. Those agents encompass agents that are useful for therapy or diagnosis (therapeutic or diagnosing agents). Those bioactive agents may be natural, synthetic, semi-synthetic or derivatives thereof and may include hydrophobic, hydrophilic, soluble and insoluble compounds. More specifically, it may be any bioactive agent useful for the treatment and/or prevention and/or diagnosis of conditions in any therapeutic area known in mammals, such as animals and humans, particularly humans. In a particular embodiment, bioactive agents according to the invention further include agents appropriate for detecting the polymer of the invention or the nanocarrier formed by the self-assembly of this polymer such as fluorescent agents, such as fluorescent probes, such as fluorescein, quantum dots, cyanine dyes Cy3® and Cy5®, Alexa Fluor® dyes, Dylight fluor® dyes, IRIS® Dyes, Seta® dyes, SeTau® dyes, SRfluor® dyes, Square® dyes, Nile red, iFLI[AMl] or carboxytetramethylrhodamine (TAMRA); chelates or cages enabling the association of nuclear magnetic resonance (NMR) tags, such as xenon or lanthanides (in particular terbium (Tb) or europium (Eu)); chelates for magnetic resonance imaging (MRI) contrast agents such as gadolinium (Gd) chelates or technetium (Tc) chelates for SPECT imaging; mass spectrometry tags such as tris(2,4,6 trimethoxyphenyl)phosphonium (TMPP) or isotope-coded tags; infrared (IR) tags; positron emission tomography (PET) tags; single-photon emission computed tomography (SPECT) tags; tritium or deuterium atoms; microscopy tags such as gold nanoparticles; quenchers such as dabsyl (dimethylaminoazobenzene sulfonic acid).

The term "alkyl" when used alone or in combination with other terms, comprises a straight or branched chain of C₁-C₂₀ alkyl which refers to monovalent alkyl groups having 1 to 20 carbon atoms. This term is exemplified by groups such as methyl, ethyl, n-propyl, i-propyl, n-butyl, s-butyl, i-butyl, t-butyl, n-pentyl, 1-ethylpropyl, 2-methylbutyl, 3-methylbutyl, 2,2-dimethylpropyl, n-hexyl, 2-methylpentyl, 3-methylpentyl, 4-methylpentyl, n-heptyl, 2-methylhexyl, 3-methylhexyl, 4-methylhexyl, 5-methylhexyl, n-heptyl, n-octyl, n-nonyl, n-decyl, tetrahydrogeranyl, n-dodecyl, n-tridecyl, n-tetradecyl, n-pentadecyl, n-hexadecyl, n-octadecyl, n-nonadecyl, and n-eicosanyl and the like. Preferably, these include C₁-C₉ alkyl, more preferably C₁-C₆ alkyl, especially preferably C₁-C₄ alkyl, which, by analogy, refers respectively to monovalent alkyl groups having 1 to 9 carbon atoms, monovalent alkyl groups having 1 to 6 carbon atoms and monovalent alkyl groups having 1 to 4 carbon atoms. Particularly, those include C₁-C₆ alkyl. Alkyl groups can be interrupted by at least one heteroatom. It may be cited the polyethyleneglycol groups of formula -(OCH₂-CH₂)n-, wherein n is from 1 to 1000, preferably from 1 to 100, in particular from 1 to 8.

The term "alkenyl" when used alone or in combination with other terms, comprises a straight chain or branched C₂-C₂₀ alkenyl. It may have any available number of double bonds in any available positions, and the configuration of the double bond may be the (E) or (Z) configuration. This term is exemplified by groups such as vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, 1-heptenyl, 1-octenyl, geranyl, 1-decenyl, 1-tetradecenyl, 1-octadecenyl, 9-octadecenyl, 1-eicosenyl, and 3, 7, 11, 15-tetramethyl-1-hexadecenyl, and the like. Preferably, these include C₂-C₈ alkenyl, more preferably C₂-C₆ alkenyl. Among others, especially preferred are vinyl or ethenyl (-CH=CH₂), n-2-propenyl (allyl, -CH₂CH=CH₂), isopropenyl, 1-propenyl, 2-methyl-1-propenyl, 1-butenyl, 2-butenyl, and 3-methyl-2-butenyl and the like.

The term "alkynyl" when used alone or in combination with other terms, comprises a straight chain or branched C₂-C₂₀ alkynyl. It may have any available number of triple bonds in any available positions. This term is exemplified by groups such as alkynyl groups that may have a carbon number of 2-20, and optionally a double bond, such as ethynyl (-C≡CH), 1-propynyl, 2-propynyl (propargyl: -CH₂C≡CH), 2-butynyl, 2-pentene-4-ynyl, and the like. Particularly, these include C₂-C₈ alkynyl, more preferably C₂-C₆ alkynyl and the like. Preferably those include C₂-C₆ alkynyl which refers to groups having 2 to 6 carbon atoms and having at least 1 or 2 sites of alkynyl unsaturation.

The term "aryl" refers to an unsaturated aromatic carbocyclic group of from 6 to 14 carbon atoms having a single ring (e.g., phenyl) or multiple condensed rings (e.g., indenyl, naphthyl). Aryl includes phenyl, naphthyl, anthryl, phenanthrenyl and the like.

The term "heteroaryl" refers to a monocyclic heteroaromatic, or a bicyclic or a tricyclic fused-ring heteroaromatic group. Particular examples of heteroaromatic groups include optionally substituted pyridinyl, pyrimidinyl, pyrazinyl, pyridazinyl, triazinyl, furanyl, thiophenyl, pyrrolyl, imidazolyl, thiazolyl, oxazolyl, isoxazolyl, dihydroisoxasolyl, triazolyl, diazinyl, tetrazinyl, pyrazolyl and naphthyl groups. In particular, an aryl group is selected from the group consisting of isoxazolyl, dihydroisoxasolyl, triazolyl, diazinyl, tetrazinyl and pyrazolyl groups.

The term "amino" refers to the group -NRR' where R and R' are independently H, "C₁-C₆ alkyl", "aryl", "heteroaryl", "C₁-C₆ alkyl aryl", "C₁-C₆ alkyl heteroaryl," "cycloalkyl," or "heterocycloalkyl," and where R and R', together with the nitrogen atom to which they are attached, can optionally form a 3-8-membered heterocycloalkyl ring.

The term "alkoxycarbonyl" refers to the group -C(O)OR where R includes "C₁-C₆ alkyl", "aryl", "heteroaryl", "aryl C₁-C₆ alkyl", "heteroaryl C₁-C₆ alkyl" or "heteroalkyl".

The term "acyl" refers to the group -C(O)R where R includes H, "C₁-C₆ alkyl," preferably "C₁-C₆ alkyl," "aryl," "heteroaryl," "C₃-C₈-cycloalkyl," "heterocycloalkyl," "aryl C₁-C₆ alkyl," "heteroaryl C₁-C₆ alkyl," "C₃-C₈-cycloalkyl C₁-C₆ alkyl" or "heterocycloalkyl C₁-C₆ alkyl", including acetyl and the like.

Unless otherwise constrained by the definition of the individual substituent, the term "substituted" refers to groups substituted with from 1 to 5 substituents selected from the group consisting of "C₁-C₆ alkyl," "C₂-C₆ alkenyl," "C₂-C₆ alkynyl," "C₃-C₈-cycloalkyl," "heterocycloalkyl," "C₁-C₆ alkyl aryl," "C₁-C₆ alkyl heteroaryl," "C₁-C₆ alkyl cycloalkyl," "C₁-C₆ alkyl heterocycloalkyl," "cycloalkyl C₁-C₆ alkyl," "heterocycloalkyl C₁-C₆ alkyl," "amino," "aminosulfonyl," "ammonium," "alkoxy," "acyl amino," "amino carbonyl," "aryl," "aryl C₁-C₆ alkyl," "heteroaryl," "heteroaryl C₁-C₆ alkyl," "sulfinyl," "sulfonyl," "sulphonamide", "alkoxy," "alkoxy carbonyl," "carbamate," "sulfanyl," "halogen," "carboxy," trihalomethyl, cyano, hydroxy, mercapto, nitro, and the like.

The term "C₈ cycloalkynyl" refers to a unsaturated carbocyclic group of 8 carbon atoms having a triple bond and being a single ring (e.g., cyclooctyne, monofluorinated cyclooctyne, difluorocyclooctyne, nonfluorocyclooctyne, aryl-less cyclooctyne, and dimethoxyazacyclooctyne) or multiple condensed rings (e.g., dibenzocyclooctyne (DBCO), biarylazacyclooctynone, dibenzoazacyclooctyne, and bicyclononyne) or those as described in

Debets et al., 2011, Accounts for Chemical Research, 44(9), 805-815*.*

The term "C₈ heterocycloalkynyl" refers to a C₈ cycloalkynyl group according to the definition above, in which up to 3 carbon atoms are replaced by heteroatoms chosen from the group consisting of O, S, NR, R being defined as hydrogen or methyl. Cg heterocycloalkynyl includes optionally substituted aza-dibenzocyclooctynyl and the like.

The term "pharmaceutically acceptable" refers to a carrier comprised of a material that is not biologically or otherwise undesirable and not especially toxic.

The term "carrier" refers to any component present in a pharmaceutical formulation other than the active agent and thus includes diluents, binders, lubricants, disintegrants, fillers, coloring agents, wetting or emulsifying agents, pH buffering agents, preservatives and the like.

As used herein, "treatment" and "treating" and the like generally mean obtaining a desired pharmacological and/or physical and/or physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions such as improvement or remediation of damage.

The term "subject" as used herein refers to mammals. For examples, mammals contemplated by the present invention include human, primates, and domesticated animals such as cattle, sheep, pigs, horses and particularly racehorses, laboratory rodents and the like.

The term "efficacy" of a treatment according to the invention can be measured based on changes in the course of disease in response to a use according to the invention.

### Polymers according to the invention

According to one embodiment, is provided a bi-functionalizable amphiphilic di-block co-polymer comprising:
- a hydrophilic polymer;
- a biodegradable hydrophobic polyester; and
- two functionalizable groups at each end of the said amphiphilic di-block co-polymer, wherein said functionalizable groups can be conjugated by an addition reaction (e.g., click) in aqueous media to another reacting moiety suitable for click chemistry, or any pharmaceutically acceptable salts thereof.

According to a particular embodiment, is provided a bi-functionalizable amphiphilic di-block co-polymer of Formula (I):

**F₁**-R₁-R₂-R₃-**A**-R₄-R₅-R₆-**B**-R₇-R₈-R₉-**F₂** **(I)**

wherein F₁ and F₂ are two functionalizable groups which can be conjugated by addition reaction, in particular, click reaction in aqueous media to another reacting moiety suitable for click chemistry; R₂, R₅, and R₈, are linking groups; R₁, R₃, R₄, R₆, R₇, and R₉ are optional spacing groups; **A** is a hydrophilic polymer and **B** is a biodegradable hydrophobic polyester or any pharmaceutically acceptable salts thereof.

According to a particular embodiment, is provided a bi-functionalizable amphiphilic di-block co-polymer of the invention, wherein **F₁** and **F₂** are two functionalizable groups independently selected from the following group: Cg cycloalkynyls, Cg heterocycloalkynyls C₂-C₆ alkynes, azides, carboxylic acids, thiols, maleimide, biotin, avidin, halogen, amines, acrylates, active esters, epoxides, folic acid, hydrazides, nitrophenyl carbonate, orthopyridyl disulfide, protected amines (Fmoc, tBOC), vinylsulfone, sulfonate (tosyl, mesyl) and silanes.

According to a particular embodiment, is provided a bi-functionalizable amphiphilic di-block co-polymer of the invention, wherein the optional spacing groups R₁, R₃, R₄, R₆, R₇, and R₉ can be independently present or absent. When present, R₁, R₃, R₄, R₆, R₇, and R₉ are independently selected from optionally substituted C₁-C₆ alkyl, optionally substituted C₂-C₈ alkenyl or optionally substituted C₂-C₈ alkynyl groups (optionally interrupted by at least one heteroatom selected among Si, O, N and S); optionally substituted aryl groups; optionally substituted amino acid; optionally substituted optionally substituted peptidic sequence; optionally substituted C₁-C₆ alkoxy groups; and optionally substituted amino groups, wherein the optional substituting groups are selected from the following list: halogens (e.g., F, Cl, Br and I atoms, preferably, a halogen is a Cl or Br atom); hydroxylamine (-ONH₂) group, hydrazine (-NH-NH₂) group; nitro (-NO₂); azido (-N₃) group, diazonium (-N₂⁺) group, optionally in presence of a counterion, alkyl- or optionally substituted alkoxycarbonyl, optionally substituted acyl; hydroxyl (-OH) group, boronic acid -B(OR")₂ group; wherein R" is a hydrogen atom or an alkyl group; phosphine or phosphonium groups; isocyanate (-N=C=O) or isothiocyanate (-N=C=S) group; chlorosulfonyl (-SO₂Cl) group; O-C(=O)-C(N₂)-CF₃ group or a -C(=O)-C(N₂)-CF₃ group; activated esters, such as -C(=O)-NHS, wherein NHS stands for N-hydrosuccinimidyl; perfluorinated esters and acylureas; -C=C-C=N group.

According to another particular embodiment, is provided a bi-functionalizable amphiphilic di-block co-polymer of the invention, wherein the optional spacing groups R₁, R₃, R₄, R₆, R₇, and R₉ are independently absent or selected from a C₁-C₆ alkyl such as ethyl and methyl and acyl such as ethyl or methyl carbonyl.

According to a particular embodiment, R₁ is a C₁-C₆ alkyl such as methyl or ethyl.

According to another particular embodiment, R₄ is methyl.

According to another particular embodiment, is provided a bi-functionalizable amphiphilic di-block co-polymer of the invention, wherein R₉ is acyl such as ethyl or methyl carbonyl.

According to a particular embodiment, is provided a bi-functionalizable amphiphilic di-block co-polymer of the invention, wherein the optional spacing groups R₁, R₃, R₄, R₆, R₇, and R₉ are absent.

According to another particular embodiment, R₃ and R₄, are absent.

According to another particular embodiment, R₁, R₃, and R₄ are absent.

According to another particular embodiment, R₃, R₄, R₆, and R₇ are absent.

According to another particular embodiment, R₁, R₃, R₄, R₆, and R₇ are absent.

The linking groups R₂, R₅, and R₈ are independently from the groups of the following list: acetal, acid anhydride, acyl, acylal, acyloin, acylsilane, aldimine, alkoxy, amides, amines, amine oxide, azo compound, azoxy, bisthiosemicarbazone, borinic acid, boronic ester, borinic ester, carbodiimide, carbonate ester, carbonyl, carboxamide, cumulene, cyanohydrins, disulfide, enamines, enol ethers, enolate anion, enone, enyne, episulfide, epoxy, ester, ether, glycoside bonds, hemiacetal, hemiaminal, hemiketal, hydrazone, hydroxamic acid, heterocycle, imine, imide, iminium, ketal, ketone, methine, methylenedioxy, n-oxoammonium, nitroamine, nitronate, nitrone, nitrosamine, organic peroxide, orthoester, oxime, phenylene, phosphinate, phosphine oxide, phosphine, phosphodiester, phosphinite, phosphite, phosphonate, phosphonite, phosphorane, semicarbazone, silyl enol ethers, silyl ethers, sulfide, sulfonamide, sulfone, sulfoxide, thioacetal, thioamide, thioester, thioether, thioketal, thioketone, thiourea, triazene, urea, xanthate.

According to a particular embodiment, is provided a bi-functionalizable amphiphilic di-block co-polymer of the invention, wherein the linking groups are independently selected from an ester and an amide group.

According to a particular embodiment, R₂ is an amide group.

According to a particular embodiment, R₅ is an ester group.

According to a particular embodiment, R₈ is an ester group.

According to a particular embodiment, the said biodegradable hydrophobic polyester is selected from polylactic acid (PLA), poly lactic-co-glycolic acid (PLGA) and poly-ε-caprolactone (PCL) or a derivative thereof.

According to a further particular embodiment, the said biodegradable hydrophobic polyester is polylactic acid (PLA) or a derivative thereof.

According to a particular embodiment, the said biodegradable hydrophobic polyester is polylactic acid (PLA) or a derivative thereof of about 200 to 120,000 Da and in particular from about 4,000 to 20,000 Da.

According to another further particular embodiment, the said biodegradable hydrophobic polyester is poly lactic-co-glycolic acid (PLGA) or a derivative thereof.

According to a particular embodiment, the said biodegradable hydrophobic polyester is poly lactic-co-glycolic acid (PLGA) or a derivative thereof having a molecular weight from about 200 to 120,000 Da, in particular from about 4,000 to 20,000 Da.

According to another further particular embodiment, the said biodegradable hydrophobic polyester is poly-ε-caprolactone (PCL) or a derivative thereof.

According to another further particular embodiment, the said biodegradable hydrophobic polyester is poly-ε-caprolactone (PCL) or a derivative thereof having a molecular weight from about 200 to 120,000 Da, in particular from about 4,000 to 20,000 Da.

According to a particular embodiment, the said hydrophilic polymer is a PEG, in particular, a PEG having a molecular weight from about 200 to 20,000 Da, in particular from about 200 to 10,000 Da, such as from about 200 to 10,000 Da, in particular from about 2,000 to 5,000 Da.

According to a particular embodiment, the said functionalizable groups are selected from biotin, alkynes such as Cg cycloalkynyl or Cg heterocycloalkynyl (e.g., DBCO), azides such as azidoacetic acid, maleimide, and halide.

According to a particular embodiment, is provided a bi-functionalizable amphiphilic di-block co-polymer is selected from the following group:
- DBCO-PLA-PEG2,000-biotin polymer;
- N₃-PLA-PEG2,000-biotin polymer;
- DBCO-PLA-PEG5,000-maleimide polymer;
- Br-PLA-PEG2,000-maleimide polymer;
- DBCO-PLA-PEG2,000-maleimide polymer;
- Br-PLA-PEG176-sulfo-DBCO polymer;
- DBCO-PLGA-PEG2,000-biotin polymer; and
- DBCO-PCL-PEG2,000-biotin polymer or any pharmaceutically acceptable salts thereof.

According to another aspect, is provided a conjugate comprising a bi-functionalizable amphiphilic di-block co-polymer of the invention, said conjugate being of Formula (II):

**F₃**-R₁-R₂-R₃-**A**-R₄-R₅-R₆-**B**-R₇-R₈-R₉-**F₄** **(II)**

wherein F₃ and F₄ are moieties resulting from the coupling of a bioactive agent with a functionalizable group F₁ and F₂ of the invention, respectively through an addition reaction (e.g., "click, nucleophilic substitution") or from the interaction of a functionalizable group F₁ and F₂ of the invention with a reacting group of a bioactive agent, said reacting group having a specific affinity for said functionalizable group F₁ and F₂; R₁, R₂, R₃, R₄, R₅, R₆, R₇, R₈, and R₉; **A** and **B** are as described herein or any pharmaceutically acceptable salts thereof.

According to a particular embodiment, is provided a bi-functionalizable amphiphilic di-block co-polymer of the invention, wherein **F₁** and **F₂** are two functionalizable groups independently selected from the following group: Cg cycloalkynyls, Cg heterocycloalkynyls C₂-C₆ alkynes, azides, carboxylic acids, thiols, maleimide, biotin, avidin, halogen, amines, acrylates, active esters, epoxides, folic acid, hydrazides, nitrophenyl carbonate, orthopyridyl disulfide, protected amines (Fmoc, tBOC), vinylsulfone, sulfonate (tosyl, mesyl) and silanes.

According to a particular embodiment, pharmaceutically acceptable salts of copolymers or conjugates of the invention comprise such as sodium, potassium, ammonium, magnesium, and calcium.

According to a particular embodiment, a bi-functionalizable amphiphilic di-block co-polymer of the invention has an average molecular weight from about 300 to 1,000,000 Da, such as from about 3,000 to 80,000 Da.

According to a particular embodiment, one of the advantages of the bi-functionalizable amphiphilic di-block co-polymers according to the invention is that they allow providing two functional groups on the resulting nanocarriers without the need to use two different mono-functionalizable co-polymers. This has a significant impact on the follow-up and the characterization of nanocarriers because it can be stated with certainty that the two functions are part of the same nanocarrier.

### Preparation of polymers and conjugates according to the invention

The bi-functionalizable amphiphilic di-block co-polymers according to the invention can be prepared from readily available starting materials in two steps, according to the following general methods and procedures. It will be appreciated that where typical or preferred experimental conditions (i.e., reaction temperatures, time, moles of reagents, solvents, etc.) are given, other experimental conditions can also be used unless otherwise stated. Optimum reaction conditions may vary with the particular reactants or solvents used, but such conditions can be determined by the person skilled in the art, using routine optimization procedures.

The first step comprises a ring opening polymerization (ROP) of a biodegradable hydrophobic polyester precursor (e.g., lactide, lactide and glycolide or caprolactone) initiated by precursors compounds containing -OH or -NH₂ functions, in the presence of a catalyst, either for first coupling a functionalizable hydrophilic polymer to a hydrophobic polyester (Step 1, Schemes 1a-c) or first coupling a functionalizable group to a hydrophobic polyester (Step 1, Schemes 2a-c). This step could be done either in standard conditions or in a microwave reactor. The second step comprises an esterification reaction between the free alcohol obtained from step one polymer and a carboxylic acid (Schemes 1d-1f or Schemes 2d to 2f, step 2). Finally, the bi-functionalizable amphiphilic polymers are purified, dried under reduced pressure, lyophilized, and stored at 4 °C. A general synthetic approach for obtaining a bi-functionalizable amphiphilic di-block co-polymer of the invention can be to react a mono-functionalizable hydrophilic polymer with a precursor of a hydrophobic polyester under ROP conditions and then to functionalize the non-functionalized hydrophobic polyester end on the so-obtained mono-functionalizable amphiphilic di-block co-polymer by esterification according to Scheme 1 below:

### a) Step 1 - ROP

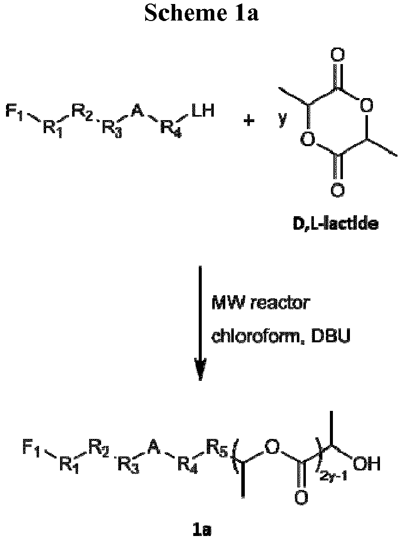

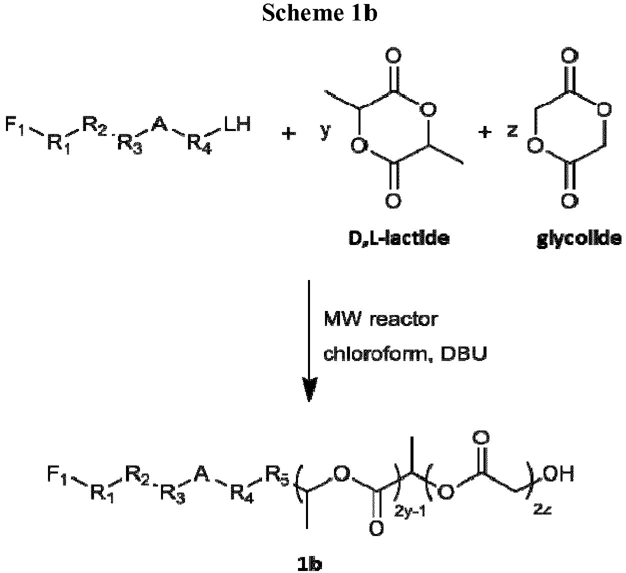

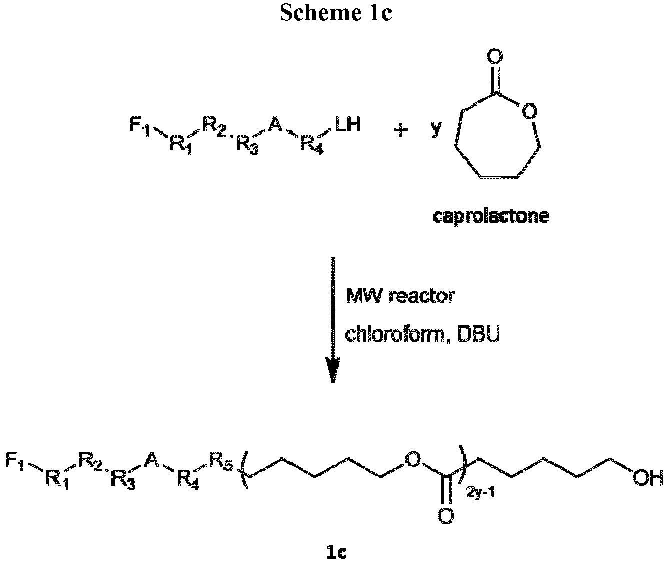

A solution of functionalizable compound (F₁-R₁-R₂-R₃-**A**-R₄-LH) (wherein L is either O or NH) (1 eq) as initiator, cyclic esters as precursors of a hydrophobic polyester such as lactide, lactide and glycolide, or caprolactone, and 1,8-diazabicyclo(5.4.0)undec-7-ene (DBU) as a catalyst (2.5 mol% relative to cyclic ester) in CHCl₃ is irradiated in a microwave reactor (Biotage® Initiator+, Uppsala, Sweden) at 75 °C for 5 min under nitrogen atmosphere. The resulting mixture is poured in an aqueous solution of HCl 0.1 M and extracted with CH₂Cl₂, washed with brine and water. Hexane is then added to the organic layer, and the precipitate is centrifuged for 5 min at 3,000 x *g*. After 3 cycles of dissolution of the pellet in a mixture of CH₂Cl₂/MeOH (8:2), precipitation in hexane and centrifugation for 5 min at 3,000 x *g*, the resulting pellet is precipitated in hexane and dried under reduced pressure to give a monofunctionalizable intermediate F₁-R₁-R₂-R₃-**A**-R₄-R₅-**B**-OH **(intermediate 1)** when R₆ and R₇ are absent such as illustrated for as F₁-R₁-R₂-R₃-**PEG-**R₄-R₅-**PLA**-OH, F₁-R₁-R₂-R₃-**PEG**-R₄-R₅-**PLGA**-OH and F₁-R₁-R₂-R₃-**PEG**-R₄-R₅-**PCL**-OH (intermediates **1a- 1c).**

### b) Step 2- esterification

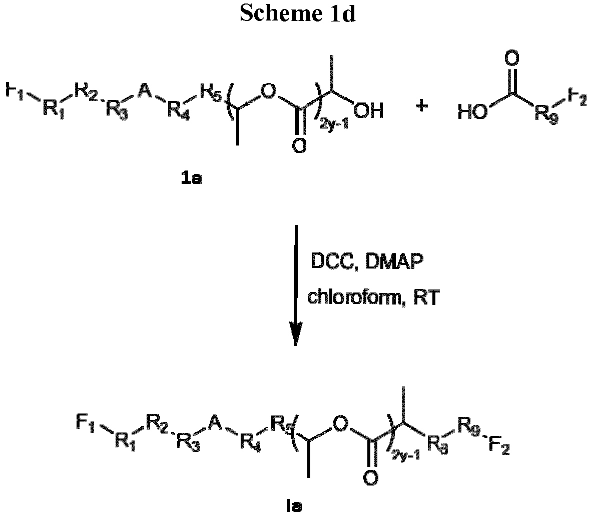

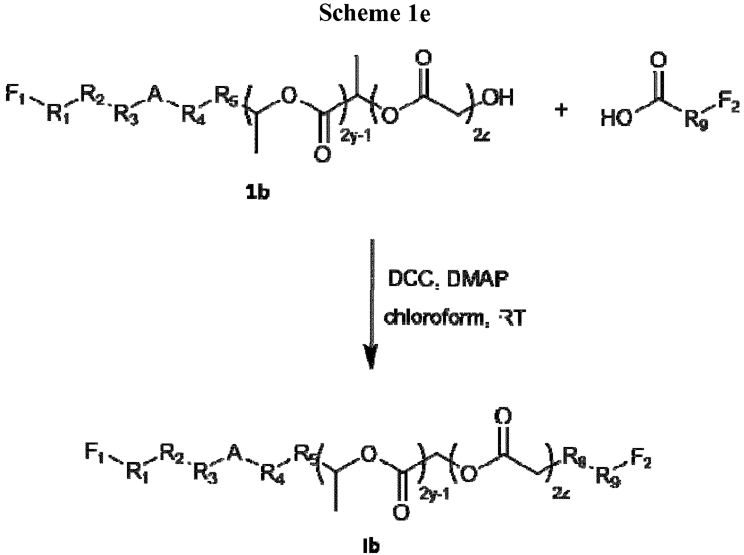

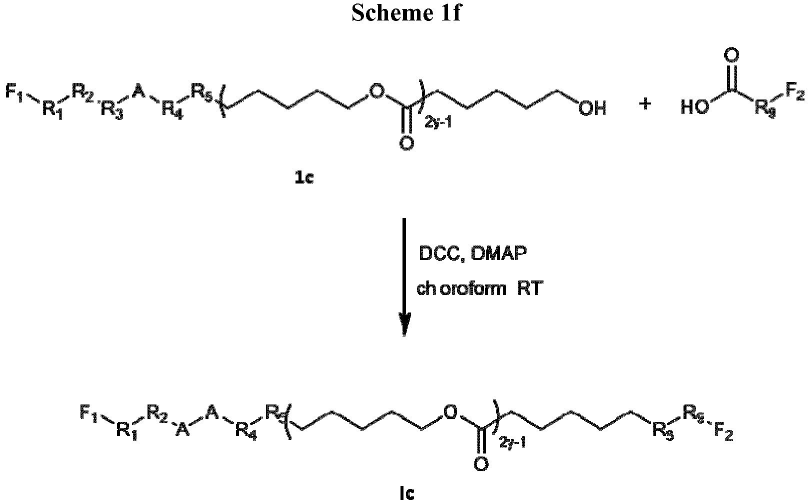

A solution of a monofunctionalizable intermediate F₁-R₁-R₂-R₃-**A**-R₄-R₅-**B**-OH **(intermediate 1) such as 1a, 1b or 1c** (1 eq), a functionalizable carboxylic acid intermediate F₂-R₉-COOH (1 eq), *N*,*N*'-dicyclohexylcarbodiimide (DCC) (1.1 eq) and 4-dimethylaminopyridine (DMAP) (10 mol% relative to intermediate **1**) in CHCl₃ is stirred at room temperature for 4 h. The reaction mixture is poured into water and extracted with CH₂Cl₂, washed with brine and water. Hexane is then added to the organic layer, and the precipitate is centrifuged for 5 min at 3,000 x *g*. After 3 cycles of dissolution of the pellet in a mixture of CH₂Cl₂/MeOH (8:2), precipitation in hexane and centrifugation for 5 min at 3,000 x *g*, the resulting pellet is precipitated in hexane and dried under reduced pressure to give a bi-functionalizable amphiphilic di-block co-polymer of the invention, illustrated for F₁-R₁-R₂-R₃-**PEG**-R₄-R₅-**PLA**-R₈-R₉-F₂, F₁-R₁-R₂-R₃-**PEG**-R₄-R₅-**PLGA**-R₈-R₉-F₂, and F₁-R₁-R₂-R₃-**PEG**-R₄-R₅-**PCL**-R₈-R₉-F₂) (Compounds **Ia-Ic).** Micelles are then formed instantly in an aqueous medium. The starting functionalizable compound (F₁-R₁-R₂-R₃-**A**-R₄-LH) and functionalizable carboxylic acid (F₂-R₉-COOH) might be commercially available or prepared by according to a standard procedure known to the skilled person.

An alternative general synthetic approach for obtaining a bi-functionalizable amphiphilic di-block co-polymer of the invention is to react a functionalizable group to a hydrophobic polyester under ROP conditions and then to functionalize the non-functionalized hydrophobic polyester end on the so-obtained mono-functionalizable hydrophobic polymer to a mono-functionalizable hydrophilic polymer by esterification according to Schemes 2a-2f below:

### a) Step 1 - ROP

A solution of functionalizable compound (F₂-R₉-LH) (wherein L is either O or NH) (1 eq) as initiator, cyclic esters as precursors of a hydrophobic polyester such as lactide, lactide and glycolide, or caprolactone as cyclic esters, and 1,8-diazabicyclo(5.4.0)undec-7-ene (DBU) as a catalyst (2.5 mol% relative to cyclic ester) in CHCl₃ is irradiated in a microwave reactor (Biotage® Initiator+, Uppsala, Sweden) at 75 °C for 5 min under nitrogen atmosphere. The resulting mixture is poured in an aqueous solution of HCl 0.1 M and extracted with CH₂Cl₂, washed with brine and water. Hexane is then added to the organic layer, and the precipitate is centrifuged for 5 min at 3,000 x *g*. After 3 cycles of dissolution of the pellet in a mixture of CH₂Cl₂/MeOH (8:2; v/v), precipitation in hexane and centrifugation for 5 min at 3,000 x *g*, the resulting pellet is precipitated in hexane and dried under reduced pressure to give a monofunctionalizable hydrophobic polymer intermediate F₂-R₉-R₈-**B**-OH **(intermediate 2)** when R₆ and R₇ are absent such as illustrated for F₂-R₉-R₈-**PLA**-OH, F₂-R₉-R₈-**PLGA-**OH and F₂-R₉-R₈-**PCL**-OH (intermediates **2a- 2c).**

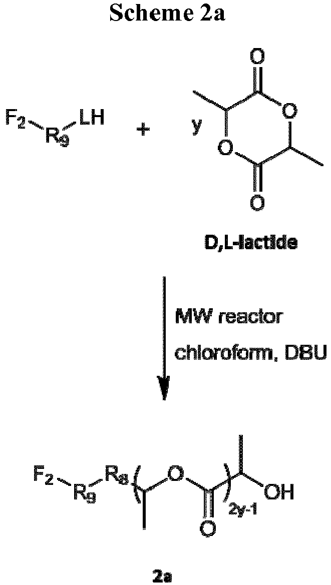

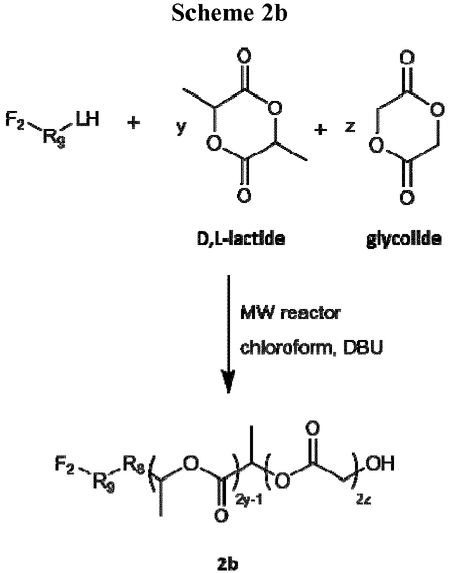

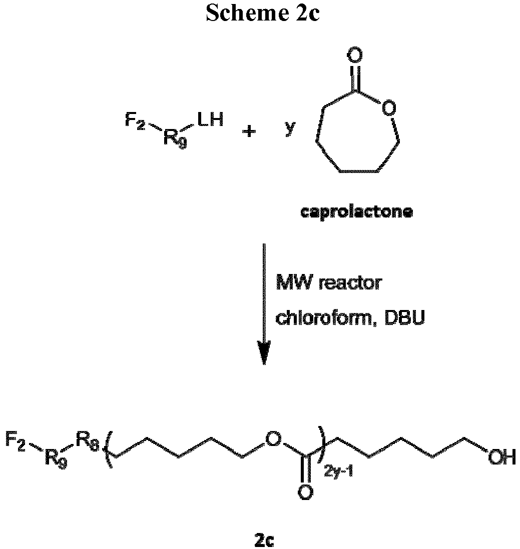

### b) Step 2- esterification

A solution of a monofunctionalizable intermediate F₂-R₉-R₈-**B**-OH **(intermediate 2) such as 2a, 2b or 2c** (1 eq), a functionalizable hydrophilic polymer (F₁-R₁-R₂-R₃-**A**-R₄-COOH) (1 eq), *N*,*N*'-dicyclohexylcarbodiimide (DCC) (1.1 eq), and 4-dimethylaminopyridine (DMAP) (10 mol % relative to **2**) in CHCl₃ was stirred at room temperature for 4 h. The reaction mixture was poured into water and extracted with CH₂Cl₂, washed with brine and water. Hexane was then added to the organic layer, and the precipitate was centrifuged for 5 min at 3,000 x *g*. After 3 cycles of dissolution of the pellet in a mixture of CH₂Cl₂/MeOH (8:2), precipitation in hexane and centrifugation for 5 min at 3,000 x *g*, the resulting pellet was precipitated in hexane and dried under reduced pressure to give a bi-functionalizable amphiphilic di-block co-polymer of the invention, illustrated for F₁-R₁-R₂-R₃-**PEG**-R₄-R₅-PLA-R₈-R₉-F₂, F₁-R₁-R₂-R₃-**PEG**-R₄-R₅-**PLGA**-R₈-R₉-F₂ and F₁-R₁-R₂-R₃-**PEG**-R₄-R₅-**PCL**-R₈-R₉-F₂) (Compounds **Ia- Ic)**. Micelles are then formed instantly in an aqueous medium. Alternatively, in the second step, the esterification could be replaced by an alkylation with a halogen derivative. Another alternative would be to modify the alcohol obtained from the first step into a ketone (for PLA or PLGA) or aldehyde (for PLGA or PCL) and then react it with an amine to achieve a polymer of the invention. Other alternatives include modifying the alcohol obtained from the first step into a halogenated derivative or with a leaving group such as tosylate or mesylate and then react with a nucleophilic group.

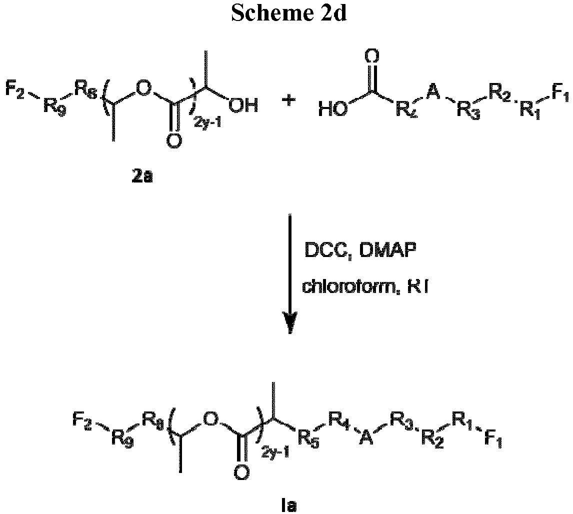

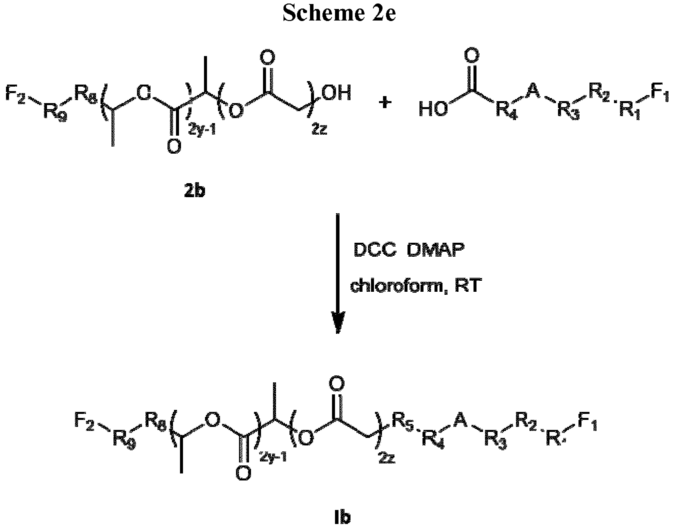

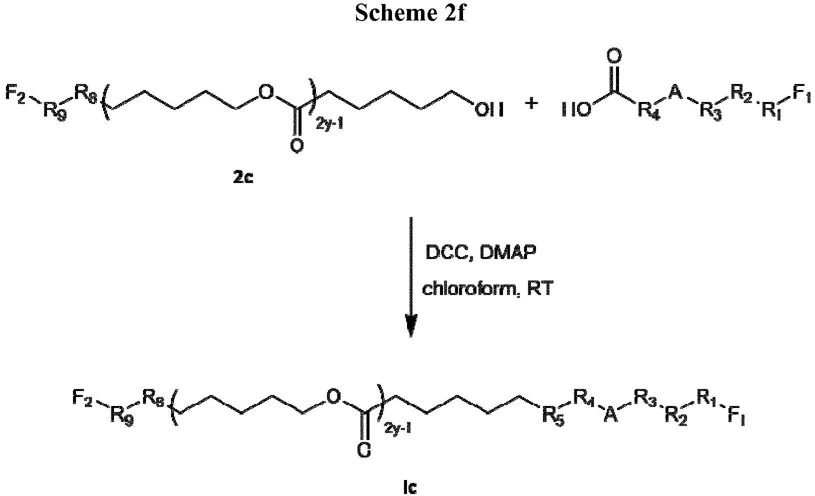

Another alternative method would be to conjugate a functionalizable hydrophilic polymer (A) to a biodegradable hydrophobic polyester (B) according to the invention, by addition (Scheme 3a) and then to introduce a functionalizable group F2 on the resulting co-polymer (Scheme 3b) as illustrated below:

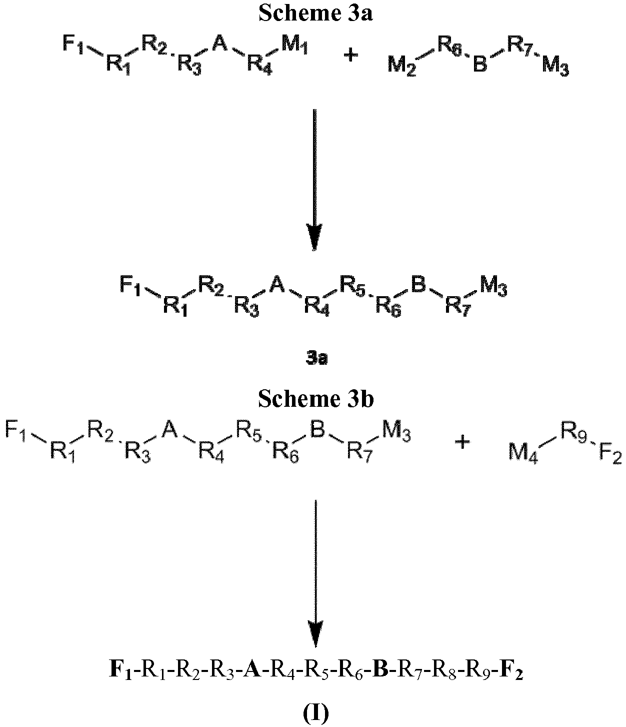

Wherein M₁, M₂, M₃, and M₄ are functionalizable groups independently selected from the following group: -OH, C8 cycloalkynyls, C8 heterocycloalkynyls C2-C6 alkynes, azides, carboxylic acids, thiols, maleimide, halogen, amines, acrylates, active esters, epoxides, folic acid, hydrazides, nitrophenyl carbonate, orthopyridyl disulfide, protected amines (Fmoc, tBOC), vinylsulfone, sulfonate (tosyl, mesyl) and silanes. Additionally, M₁ reacts with M₂ and M₃ reacts with M₄. The reaction between M₁ and M₂, and M₃ and M₄ results in R₅ and R₈ respectively as defined herein. The starting material M₂-R₆-B-R₇-M₃ can be either commercially available or synthesized by standard methods.

Therefore, the corresponding optional spacing groups and linking groups R₁, R₂, R₃, R₄, R₅, R₆, R₇, R₈, and R₉ should be adapted accordingly on the starting material for achieving a polymer of the invention *via* those routes of synthesis.

In the case of a hydrophilic biocompatible polymer having lateral chains such as PHEA, PHEG or PGA, a step of lateral chain protection is necessary before reacting the polymer chain in the first and second steps described above.

According to a particular embodiment, is provided a process for the preparation of a bi-functionalizable amphiphilic di-block co-polymer according to the invention, said process comprising the steps of:
a) Providing a precursor of a biodegradable hydrophobic polyester **B** (e.g., lactide, lactide, and glycolide or caprolactone);
b) Reacting the said precursor with a compound F₁-R₄-R₂-R₃-**A**-R₄-LH to lead to an intermediate monofunctionalizable amphiphilic di-block co-polymer F₁-R₁-R₂-R₃-**A-**R₄-R₅-R₆-**B**-R₇-OH **(intermediate 1);**
c) Reacting the said intermediate 1 with a functionalizable carboxylic acid of formula F₂-R₉-COOH **(intermediate 3)** under esterification condition to lead to a bi-functionalizable amphiphilic di-block co-polymer according to the invention.

In another particular embodiment, is provided a process for the preparation of a bi-functionalizable amphiphilic di-block co-polymer according to the invention, said process comprising the steps of:
a) Providing a precursor of a biodegradable hydrophobic polyester **B** (e.g., lactide, lactide, and glycolide or caprolactone);
b) Reacting the said precursor with a functionalizable alcohol of formula F₂-R₉-LH to lead to an intermediate monofunctionalizable hydrophobic polymer intermediate co-polymer F₂-R₉-R₈-R₇-**B**-R₆-OH **(intermediate 2);**
c) Reacting the said intermediate 2 with a functionalizable hydrophilic polymer F₁-R₁-R₂-R₃-**A**-R₄-COOH under esterification condition to lead to a bi-functionalizable amphiphilic di-block co-polymer according to the invention.

Other syntheses can be used to obtain bi-functionalizable amphiphilic di-block co-polymer of the invention starting from conventional or functionalized commercially available hydrophobic polymer. However, these syntheses are less straightforward and need more steps (e.g., protection/deprotection, coupling reactions) to arrive at a di-block co-polymer of the invention.

According to a particular aspect, conjugates comprising a bi-functionalizable amphiphilic di-block co-polymer according to the invention and at least one bioactive agent can be prepared from the bi-functionalizable amphiphilic di-block co-polymers of the invention using copper-free "click" chemistry or other standard addition procedures and the following general methods and procedures. It will be appreciated that where typical or preferred experimental conditions (i.e., reaction temperatures, time, moles of reagents, solvents, etc.) are given, other experimental conditions can also be used unless otherwise stated. A general synthetic approach for obtaining conjugates of Formula (II) can be reacting a functionalizable amphiphilic di-block co-polymer of the invention with a reactive group from a bioactive agent wherein said the reactive group is capable of participating in a "click chemistry" reaction.

According to an aspect of the invention is provided a method for the preparation of a bi-functionalized conjugate polymer of the invention comprising the steps of:
(a) providing a bi-functionalizable amphiphilic di-block co-polymer of the invention;
(b) providing a first bioactive agent having at least one first functional group complementary to one of the two functionalizable groups on the said bi-functionalizable amphiphilic di-block co-polymer and capable of participating in an addition reaction such as a "click chemistry" reaction with such first functionalizable group;
(c) reacting the said at least one first functional group of the bioactive agent with the said first functionalizable group on the bi-functionalizable amphiphilic di-block co-polymer *via* a "click chemistry" reaction to obtain a monofunctionalized conjugate polymer;
(d) providing a second bioactive agent having at least one functional group complementary to the second functionalizable group on the bi-functionalizable amphiphilic di-block co-polymer of the invention and capable of participating in a "click chemistry" reaction with such second functionalizable group; and
(e) reacting the said at least one functional group of the second bioactive agent with the said at second functionalizable group on the bi-functionalizable amphiphilic di-block co-polymer *via* a "click chemistry" reaction to obtain a bi-functionalized conjugate polymer of the invention; and
(f) isolating the bi-functionalized conjugate polymer.

According to a further particular embodiment, steps b) and c) and d) and e) respectively can be carried out concomitantly.

According to a further particular embodiment, a method for the preparation of a bi-functionalized conjugate polymer of the invention may comprise further steps of physically dispersing and/or covalently linking and/or adding at least one bioactive agent in the polymer composition obtained in step c) or e).

According to a further particular embodiment, a method for the preparation of a bi-functionalized conjugate polymer of the invention may advantageously be conducted at the time of the formation of micelles such as illustrated in Example 4.

Alternatively, a bi-functionalized conjugate polymer of the invention can be achieved through the interaction of each of the functionalizable group F₁ and F₂ of a bi-functionalizable amphiphilic di-block co-polymer of the invention with a reacting group from a bioactive agent having a specific affinity for said functionalizable group F₁ and F₂.

### Compositions

The invention provides pharmaceutical or therapeutic agents as compositions, or medical devices comprising thereof and methods for treating a subject, preferably a mammalian subject, and most preferably a human subject, who is suffering from or at risk of suffering from a medical disorder.

Bi-functionalizable amphiphilic di-block co-polymer or conjugates thereof of the invention spontaneously form micelles. However, nanoparticles can be obtained with bi-functionalizable amphiphilic di-block co-polymer or conjugates thereof of the invention by standard methods such as nanoprecipitation, solvent evaporation, double emulsification, emulsions, coacervation, salting out or dialysis.

In a particular embodiment, the invention provides a pharmaceutical formulation comprising at least one conjugate or composition according to the invention for use as a medicament or as a laboratory or diagnosis tool.

In another particular embodiment, compositions of the invention are parenteral formulations.

In a particular embodiment, compositions of the invention are injectable formulations, such as parenteral, intravenous, intra-articular, intra-arterial, intravenous, intrasynovial, intradermal, subdermal, submucosal, intravitreous, interstitial and intramuscular formulations.

In another particular embodiment, compositions of the invention are oral formulations.

In another particular embodiment, compositions of the invention are topical formulations.

In another particular embodiment, compositions of the invention are ophthalmic formulations.

Alternatively, the invention provides compositions that can be used in another mammal thus humans for the same uses as described above.

In particular, an embodiment provides compositions comprising nanoassemblies, such as micelles, nanoparticles and the like, of the bi-functionalizable amphiphilic di-block co-polymer of the invention having a mean size ranging from about 5 to 900 nm and about 10 and 300 nm.

According to another particular embodiment, compositions of the invention can be in the form of a polymersome, capsule, nano-container or micro-container.

Compositions of this invention may further comprise one or more pharmaceutically acceptable additional ingredient(s) such as alum, stabilizers, antimicrobial agents, buffers, coloring agents, flavoring agents, adjuvants, and the like.

The compositions according to the invention, together with a conventionally employed adjuvant, carrier, diluent or excipient may be placed into the form of pharmaceutical compositions and may be employed as solids, such as tablets or filled capsules, or liquids such as solutions, suspensions, ointments, emulsions, elixirs, or capsules filled with the same, films or gels, all for oral use. The compositions may also be formulated as a dry or lyophilized product for subsequent reconstitution in an aqueous medium such as water or another suitable vehicle before use.

Compositions of this invention as liquid formulations including, but not limited to, aqueous or oily suspensions, solutions, emulsions, syrups, and elixirs.

Such liquid preparations may contain additives including, but not limited to, suspending agents, emulsifying agents, non-aqueous vehicles, and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.

Further materials, as well as formulation processing techniques and the like, are set out in

The Science and Practice of Pharmacy (Remington: The Science & Practice of Pharmacy), 22nd Edition, 2012, Lloyd, Ed. Allen, Pharmaceutical Press, which is incorporated herein by reference.

Solid compositions of this invention may be in the form of tablets or lozenges formulated conventionally. For example, tablets and capsules for oral administration may contain conventional excipients including, but not limited to, binding agents, fillers, lubricants, disintegrants and wetting agents. Binding agents include, but are not limited to, syrup, acacia, gelatin, sorbitol, tragacanth, mucilage of starch and polyvinylpyrrolidone. Fillers include, but are not limited to, lactose, sugar, microcrystalline cellulose, maize starch, calcium phosphate, and sorbitol. Lubricants include, but are not limited to, magnesium stearate, stearic acid, talc, polyethylene glycol, and silica. Disintegrants include, but are not limited to, potato starch and sodium starch glycolate. Wetting agents include, but are not limited to, sodium lauryl sulfate.

Tablets may be coated according to methods well known in the art

In another particular embodiment, the compositions of the invention comprising a conjugate of the invention are in the form of a delivery system for a bioactive agent.

In another particular embodiment, the bi-functionalizable amphiphilic di-block co-polymer or the conjugate of the invention is used in the form of nano-assemblies or as nano- and microparticles or as an implant or a gel.

In one embodiment, the at least one bioactive substance may be present in an amount of between about 0.01 to 80 wt%, preferably about 0.5 to 40 wt% based on the total amount of the polymer composition of the invention.

In another particular embodiment, is provided a method of preparation of micelles of the invention comprising the steps of:
- Providing a bi-functionalizable amphiphilic di-block co-polymer or a conjugate thereof of the invention; and
- Diluting the said bi-functionalizable amphiphilic di-block co-polymer or a conjugate thereof of the invention in aqueous medium,
wherein micelles of bi-functionalizable amphiphilic di-block co-polymer or a conjugate thereof of the invention are formed spontaneously in said aqueous medium.

According to a particular aspect, nanoparticles of the invention can be useful, once loaded with an active principle, for administration and *in situ* release of the said active principle injection.

In another particular embodiment, is provided a kit for the preparation of a bi-functionalizable amphiphilic di-block co-polymer of the invention. In particular, said kit may comprise:
i) a precursor of a biodegradable hydrophobic polyester **B** (e.g., lactide, lactide, and glycolide or caprolactone); and
(ii) precursors compounds F₁-R₁-R₂-R₃-**A**-R₄-LH and F₂-R₉-COOH;
   or
(iii) precursors compounds F₂-R₉-LH and functionalizable hydrophilic polymer F₁-R₁-R₂-R₃-**A**-R₄-COOH; and
(iv) optionally a catalyst for ring opening reactions such as 1,8-diazabicyclo[5.4.0]undec-7-ene (DBU), 7-methyl-1,5,7-triazabicyclo[4.4.0]dec-5-ene (MTBD), 1,5,7-triazabicyclo[4.4.0]dec-5-ene (TBD), methane- and trifluoromethane-sulfonic acid (MsOH and TfOH), diphenyl phosphate (DPP), phosphoramidic acid or tin octoate or those described in Dove, 2012, ACS Macro Lett., 1, 1409-14121411*.*

In another particular embodiment, is provided a kit for the preparation of a conjugate of a bi-functionalizable amphiphilic di-block co-polymer of the invention, said kit comprising at least one bi-functionalizable amphiphilic di-block co-polymer of the invention in lyophilized form or in suspension in an aqueous medium.

In another particular embodiment, is provided a kit for the preparation of nanocarriers for encapsulation of material, said kit may comprise a bi-functionalizable amphiphilic di-block copolymer according to the invention.

### Mode of administration

Compositions of this invention may be administered in any manner by oral route including to the mucosal surfaces of the oral cavity including the gingiva, the floor of the oral cavity, cheeks, lips, tongue, teeth.

Compositions of this invention may also be administered in any manner by injection such as parenteral, subcutaneous injections, intra-synovial, intra-arterial, intravenous, intraarticular, intramuscular, intraocular, subdermal, submucosal and interstitial injections.

Compositions of this invention may also be administered topically to the skin, various mucous or the surface of the eye.

### Combination

According to a particular aspect, co-agents according to the invention include drugs, active pharmaceutical ingredients, fluorophores/dyes, small molecules, targeting molecules, polymers, peptides, proteins as well as bioactive agents according to the invention.

### Patients

In an embodiment, subjects according to the invention are subjects suffering or at risk of suffering from cancer, cardiovascular diseases, inherited monogenic diseases, neurological diseases, ocular pathologies, infectious diseases, infectious diseases, lung diseases, arthritic diseases, rheumatic diseases, kidney diseases, skin diseases, gastrointestinal and hepatic diseases, bone diseases, and neurological diseases.

### Use according to the invention

According to a particular embodiment, the conjugates of the invention and compositions thereof are useful for various applications such medical applications or diagnostic or theranostic applications for human or veterinary uses (such as oncological, ocular, topical, parenteral applications), in particular as delivery system for at least one bioactive agent or cell delivery system in tissue engineering, cell culture systems, including stem cell culture or administration, and the like, cosmetic applications and potentially other fields such as in food industry.

According to another particular embodiment, the conjugates of the invention and compositions thereof are useful for use in the prevention or treatment of a medical disorder, and in particular cancer, cardiovascular diseases, inherited monogenic diseases, neurological diseases, ocular pathologies, infectious diseases, infectious diseases, lung diseases, arthritic diseases, rheumatic diseases, kidney diseases, skin diseases, gastrointestinal and hepatic diseases, bone diseases, and neurological diseases.

According to another further particular aspect, are provided polymers the invention and compositions thereof for use *in vivo* drug delivery.

Examples illustrating the invention will be described hereinafter in a more detailed manner and by reference to the embodiments represented in the Figures.

### EXAMPLES

The following abbreviations refer respectively to the definitions below:
**THF** (tetrahydrofuran), **DMSO** (dimethyl sulfoxide), **MWCO** (Molecular weight cut-off).

### Example 1: Synthesis of bi-functionalizable amphiphilic diblock copolymers

Bi-functionalizable amphiphilic diblock copolymers of the invention were synthesized as described in Schemes 1 or 2 above; wherein the following commercial starting materials were used: Biotin-PEG2,000-OH, maleimide-PEG5,000-OH, biotin-PEG2,000-SH, and maleimide-PEG2,000-COOH were purchased from Creative PEGworks (Durham, NC, USA), DBCO-COOH, N₃-COOH, and sulfo-DBCO-PEG176-NH₂ were purchased from Click Chemistry Tools (Scottsdale, AZ, USA), DBCO-NH₂, D,L-lactide, glycolide, and ε-caprolactone, *N*-2-hydroxyethylmaleimide and bromohexanol were purchased from Sigma Aldrich (Saint-Louis, MO, USA).

### Preparation of DBCO-PLA-PEG2,000-biotin polymer (1)

A bi-functionalizable amphiphilic diblock copolymer of the invention, wherein the hydrophilic polymer PEG2,000 (A), the biodegradable hydrophobic polyester PLA (B) and the functionalizable groups biotin (F1) and DBCO (F2) was prepared according to Scheme 1, as described in Schemes 4 and 5 below:

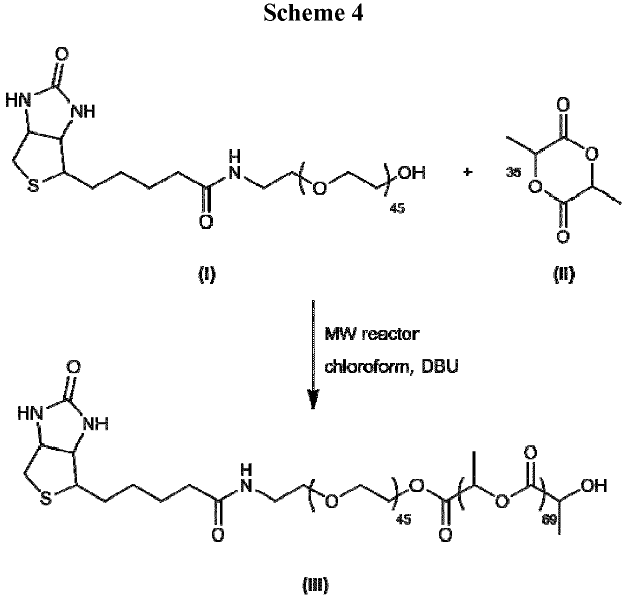

A solution of biotin-PEG2,000-OH (79 mg, 1 eq) **(i)** (starting from functionalizable alcohol F₁-R₁-R₂-R₃-**A**-R₄-LH, wherein F₁ is biotin, R₂ is an amide group, A is a PEG2,000, L is O, and the spacing groups R₁, R₃ and R₄ are absent) as initiator, D,L-lactide **(ii)** (200 mg, 35 eq) as precursor of the hydrophilic ester polymer (PLA), and 1,8-diazabicyclo(5.4.0)undec-7-ene (DBU) as a catalyst (2.5 mol% relative to D,L-lactide) in CHCl₃ (0.7 mL) was irradiated in a microwave reactor (Biotage® Initiator+, Uppsala, Sweden) at 75 °C for 5 min under nitrogen atmosphere. The resulting mixture was poured in an aqueous solution of HCl 0.1 M and extracted with CH₂Cl₂, washed with brine and water. Hexane was then added to the organic layer, and the precipitate was centrifuged for 5 min at 3,000 x *g*. After 3 cycles of dissolution of the pellet in a mixture of CH₂Cl₂/MeOH (8:2), precipitation in hexane and centrifugation for 5 min at 3,000 x *g*, the resulting pellet was precipitated in hexane and dried under reduced pressure to give the PLA-PEG2,000-biotin intermediate **(iii) (intermediate 1a** F₁-R₁-R₂-R₃-**PEG**-R₄-R₅-R₆-**PLA**-R₇-OH, wherein F₁ is biotin, R₂ is an amide group, R₅ is an ester group, and the spacing groups R₁, R₃, R₄, R₆, and R₇ are absent).

A solution of the PLA-PEG2,000-biotin intermediate **(iii)** obtained above (75 mg, 1 eq), a functionalizable carboxylic acid (F₂-R₉-COOH **(intermediate 3)** wherein F₂ is a C₈ heterocycloalkynyl, i.e., dibenzocyclooctyne and R₉ is ethyl carbonyl) DBCO-COOH (3.3 mg, 1 eq) **(iv),** *N*,*N*'-dicyclohexylcarbodiimide (DCC) (2.4 mg, 1.1 eq) and 4-dimethylaminopyridine (DMAP) (10 mol% relative to PLA-PEG2,000-biotin intermediate) in CHCl₃ (2 mL) was stirred at room temperature for 4 h. The reaction mixture was poured into water and extracted with CH₂Cl₂, washed with brine and water. Hexane was then added to the organic layer, and the precipitate was centrifuged for 5 min at 3,000 x *g*. After 3 cycles of dissolution of the pellet in a mixture of CH₂Cl₂/MeOH (8:2), precipitation in hexane and centrifugation for 5 min at 3,000 x *g*, the resulting pellet was precipitated in hexane and dried under reduced pressure to give as bi-functionalizable amphiphilic diblock copolymer of the invention, DBCO-PLA-PEG2,000-biotin polymer **(1).**

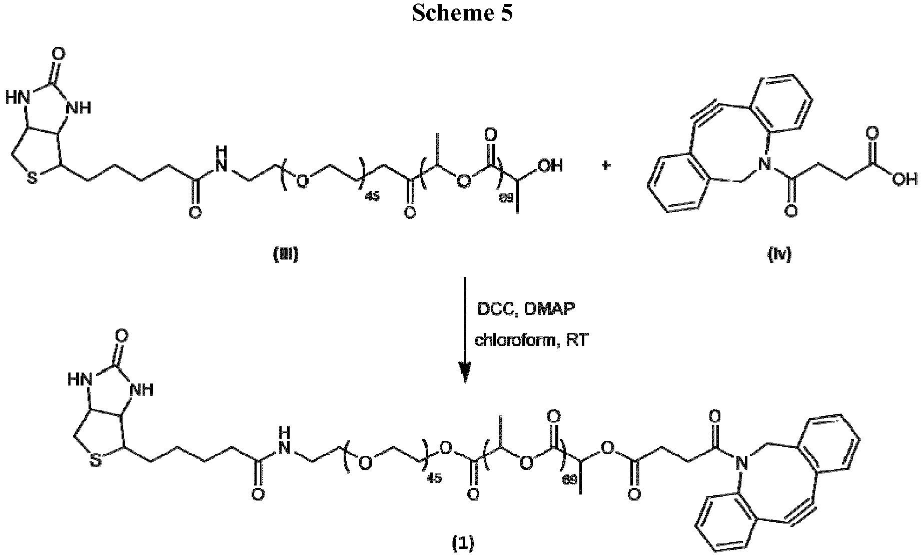

### Preparation of N3-PLA-PEG2,000-biotin polymer (2)

A bi-functionalizable amphiphilic diblock copolymer of the invention, wherein the hydrophilic polymer is a PEG2,000 (A), the biodegradable hydrophobic polyester, a PLA (B) and the functionalizable groups biotin (F₁) and N₃ (F₂) was prepared according to Scheme 1. First, a PLA-PEG2,000-biotin intermediate **(iii)** was obtained as described in Scheme 4 and then, reacted as described according to Scheme 6 below:

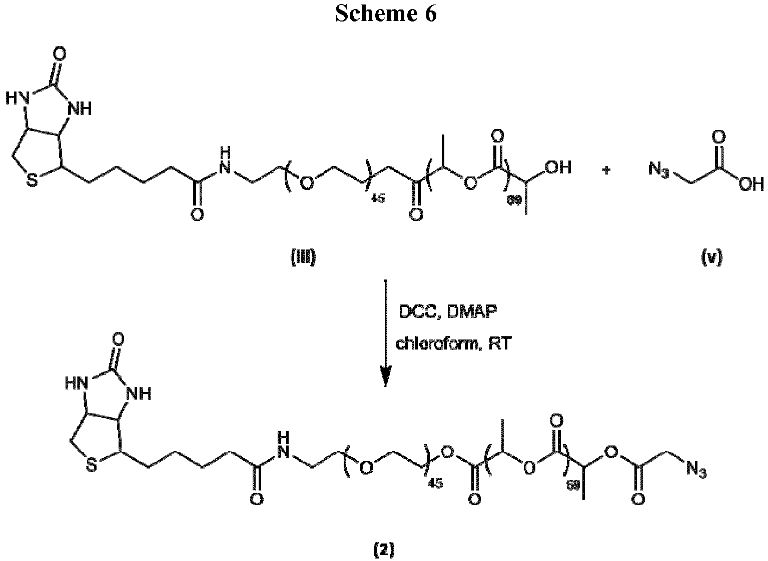

A solution of PLA-PEG2,000-biotin intermediate **(iii)** (75 mg, 1 eq), a functionalizable carboxylic acid (F₂-R₉-COOH **(intermediate 2)** wherein F₂ is an azide and R₉ is methylene) N₃-COOH (1 mg, 1 eq) **(v),** N,N'-dicyclohexylcarbodiimide (DCC) (2.4 mg, 1.1 eq) and 4-dimethylaminopyridine (DMAP) (10 mol% relative to PLA-PEG2,000-biotin intermediate) in CHCl₃ (2 mL) were reacted as described for compound **(1)** to give as bi-functionalizable amphiphilic diblock copolymer of the invention, the N₃-PLA-PEG2,000-biotin polymer **(2).**

### Preparation of DBCO-PLA-PEG5,000-maleimide polymer (3)

A bi-functionalizable amphiphilic diblock copolymer of the invention, wherein the hydrophilic polymer is a PEG5,000 (A), the biodegradable hydrophobic polyester, a PLA (B) and the functionalizable groups maleimide (F₁) and DBCO (F₂) was prepared according to Scheme 1, as described in Schemes 7 and 8 below.

A solution of maleimide-PEG5,000-OH (25 mg, 1 eq) **(vi)** as starting functionalizable alcohol (F₁-R₁-R₂-R₃-**A**-R₄-LH, wherein F₁ is maleimide and R₁ is an ethyl, R₂ is an amide group, L is O, and the spacing groups R₃ and R₄ are absent), as initiator, D,L-lactide (50 mg, 70 eq) **(ii),** and 1,8-diazabicyclo(5.4.0)undec-7-ene (DBU) as a catalyst (2.5 mol% relative to D,L-lactide) in CHCl₃ (0.5 mL) was irradiated in a microwave reactor and reacted as described above for intermediate **(iii)** to give the PLA-PEG5,000-maleimide intermediate **(vii) (intermediate 1a** F₁-R₁-R₂-R₃-**PEG**-R₄-R₅-R₆-**PLA**-R₇-OH, wherein F₁ is maleimide, R₁ is an ethyl, R₂ is an amide group, R₅ is an ester group, and the spacing groups R₃, R₄, R₆, and R₇ are absent).

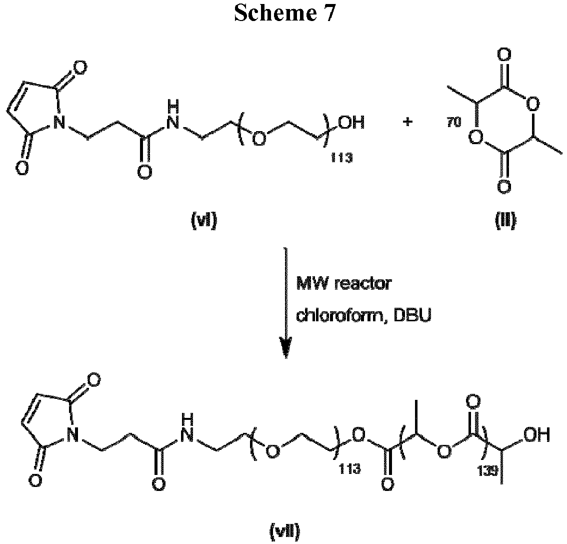

A solution of PLA-PEG5,000-maleimide intermediate **(vii)** obtained above (50 mg, 1 eq), a functionalizable carboxylic acid, DBCO-COOH **(iv)** (1 mg, 1 eq), *N,N'-*dicyclohexylcarbodiimide (DCC) (0.8 mg, 1.1 eq) and 4-dimethylaminopyridine (DMAP) (10 mol% relative to PLA-PEG5,000-maleimide intermediate) in CHCl₃ (1 mL) were reacted as described above for polymer **(1)** to give as bi-functionalizable amphiphilic diblock copolymer of the invention, the DBCO-PLA-PEG5,000-maleimide polymer **(3).**

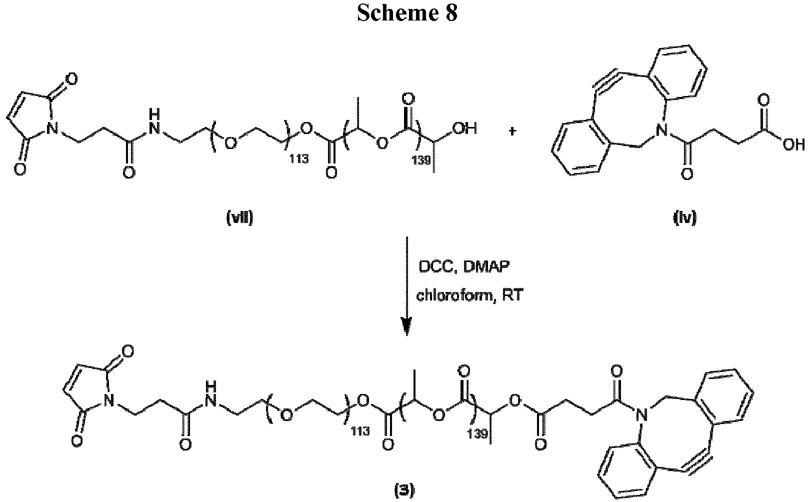

### Preparation of Br-PLA-PEG2,000-maleimide polymer (4)

A bi-functionalizable amphiphilic diblock copolymer of the invention, wherein the hydrophilic polymer is a PEG2,000 (A), the biodegradable hydrophobic polyester a PLA (B) and the functionalizable groups bromo (F₂) and maleimide (F₁) was prepared according to Scheme 2, as described in Schemes 9 and 10 below.

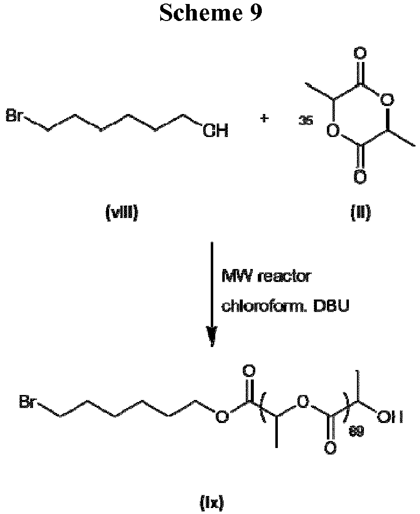

A solution of bromohexanol (13 mg, 1 eq) **(viii)** as functionalizable alcohol (F₂-R₉-LH, wherein F₂ is a bromo group, R₉ is hexyl and L is O) (1 eq) as initiator, D,L-lactide (400 mg, 35 eq) **(ii),** and 1,8-diazabicyclo(5.4.0)undec-7-ene (DBU) as a catalyst (2.5 mol% relative to D,L-lactide) in CHCl₃ (1 mL) was irradiated in a microwave reactor and reacted as described above for intermediate **(iii)** to give the Br-PLA intermediate **(ix)** (intermediate 2a F₂-R₉-R₈-R₇-**PLA**-R₆-OH, wherein F₂ is a bromo group, R₉ is hexyl, R₈ is an ester group and the spacing groups R₇ and R₆ are absent).

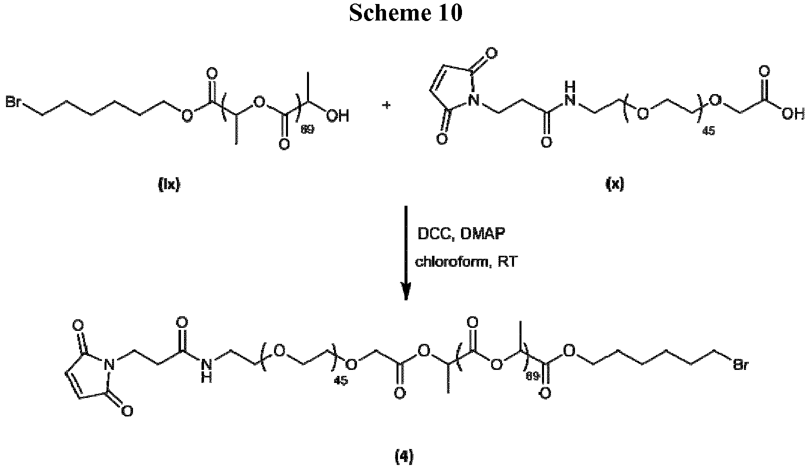

A solution of Br-PLA intermediate **(ix)** obtained above (100 mg, 1 eq), maleimide-PEG2,000-COOH (40 mg, 1 eq) **(x)** as a functionalizable hydrophilic polymer (F₁-R₁-R₂-R₃-**A**-R₄-COOH, wherein F₁ is maleimide, R₁ is an ethyl, R₂ is an amide group, R₄ is a methyl, and the spacing group R₃ is absent), *N*,*N*'-dicyclohexylcarbodiimide (DCC) (4.5 mg, 1.1 eq) and 4-dimethylaminopyridine (DMAP) (10 mol% relative to Br-PLA intermediate) in CHCl₃ (4 mL) were reacted as described above for polymer **(1)** to give as bi-functionalizable amphiphilic di-block co-polymer of the invention, the Br-PLA-PEG2,000-maleimide polymer **(4).**

### Preparation of DBCO-PLA-PEG2,000-maleimide polymer (5)

A bi-functionalizable amphiphilic diblock copolymer of the invention, wherein the hydrophilic polymer is a PEG2,000 (A), the biodegradable hydrophobic polyester, a PLA (B) and the functionalizable groups DBCO (F₂) and maleimide (F₁) was prepared according to Scheme 2, as described in Schemes 11 and 12 below:

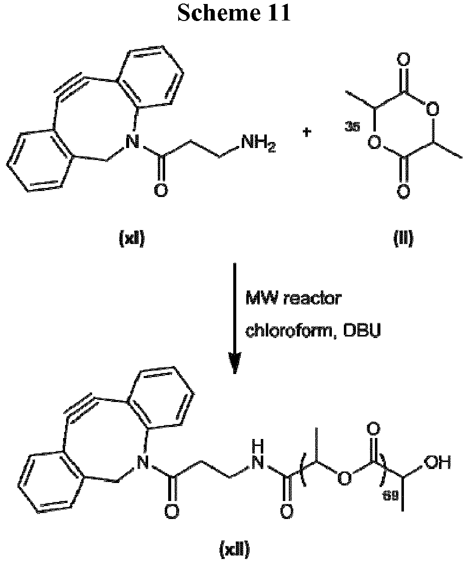

A solution of DBCO-NH₂ **(viii)** (22 mg, 1 eq) as functionalizable amine (F₂-R₉-LH wherein F₂ is a C₈ heterocycloalkyne such as dibenzocyclooctyne, R₉ is ethyl carbonyl and L is NH) as initiator, D,L-lactide **(ii)** (400 mg, 35 eq), and 1,8-diazabicyclo(5.4.0)undec-7-ene (DBU) as a catalyst (2.5 mol% relative to D,L-lactide) in CHCl₃ (1 mL) was irradiated in a microwave reactor and reacted as described above for intermediate **(iii)** to give the DBCO-PLA intermediate **(xii)** (intermediate 2a F₂-R₉-R₈-R₇-**PLA**-R₆-OH, wherein F₂ is DBCO, R₉ is ethyl carbonyl, R₈ is an amide group, and the spacing groups R₇ and R₆ are absent).

A solution of DBCO-PLA intermediate **(xii)** obtained above (100 mg, 1 eq), maleimide-PEG2,000-COOH **(x)** as a functionalizable hydrophilic polymer (F₁-R₁-R₂-R₃-**A**-R₄-COOH, wherein F₁ is maleimide, R₁ is an ethyl, R₂ is an amide group, A is PEG2,000, R₄ is a methyl, and the spacing group R₃ is absent) (40 mg, 1 eq), *N*,*N*'-dicyclohexylcarbodiimide (DCC) (4.5 mg, 1.1 eq) and 4-dimethylaminopyridine (DMAP) (10 mol% relative to DBCO-PLA intermediate) in CHCl₃ (4 mL) were reacted as described above for polymer **(1)** to give as bi-functionalizable amphiphilic di-block co-polymer of the invention, the DBCO-PLA-PEG2,000-maleimide polymer **(5).**

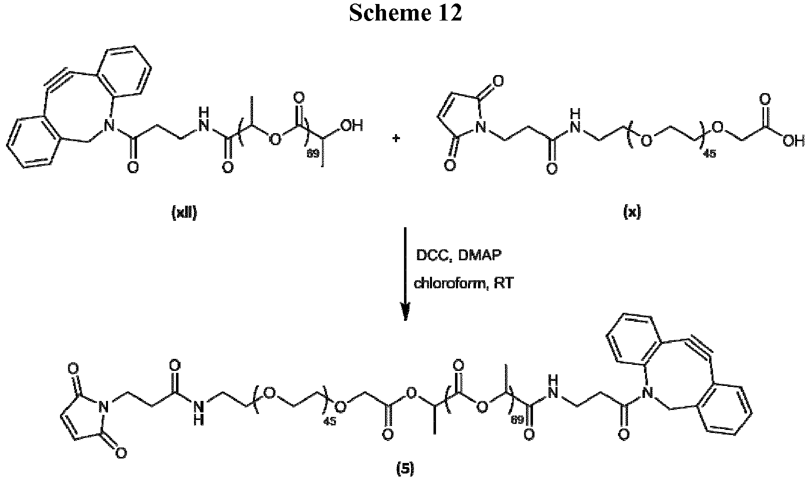

### Preparation of Br-PLA-PEG176-sulfo-DBCO polymer (6)

A bi-functionalizable amphiphilic diblock copolymer of the invention, wherein the hydrophilic polymer is a PEG176 (A), the biodegradable hydrophobic polyester, a PLA (B) and the functionalizable groups bromo (F2) and DBCO (F1) was prepared according to Scheme 1, as described in Schemes 13 and 14 below:

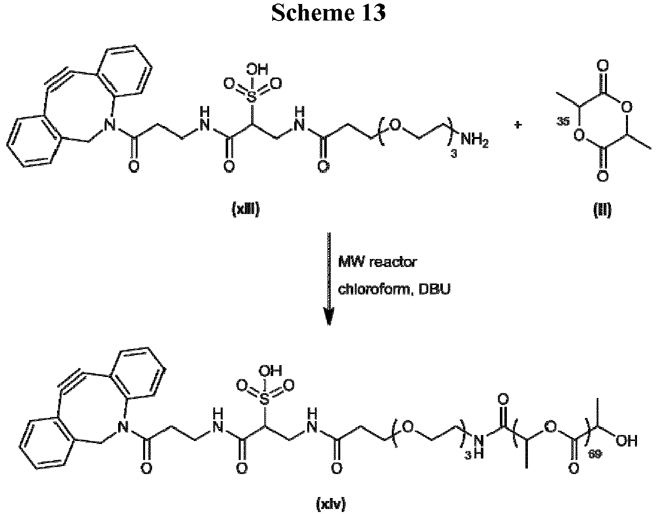

A solution of sulfo-DBCO-PEG176-NH₂ **(xiii)** as starting functionalizable alcohol (F₁-R₁-R₂-R₃-A-R₄-LH, wherein F₁ is a Cg heterocycloalkyne such as sulfo-DBCO, R₂ is an amide group, A is PEG176, L is NH, and the spacing groups R₁, R₃ and R₄ are absent) (6.7 mg, 1 eq) as initiator, D,L-lactide (50 mg, 35 eq) **(ii),** and 1,8-diazabicyclo(5.4.0)undec-7-ene (DBU) as a catalyst (2.5 mol% relative to D,L-lactide) in CHCl₃ (0.5 mL) was irradiated in a microwave reactor and reacted as described above for intermediate **(iii)** to give the sulfo-DBCO-PEG176-PLA intermediate **(xiv) (intermediate 1a** F₁-R₁-R₂-R₃-**PEG**-R₄-R₅-R₅-**PLA**-R₇-OH, wherein F₁ is sulfo-DBCO, R₂ is an amide group, R₅ is an amide group, R₁, R₃, R₄, R₆ and R₇ are absent).

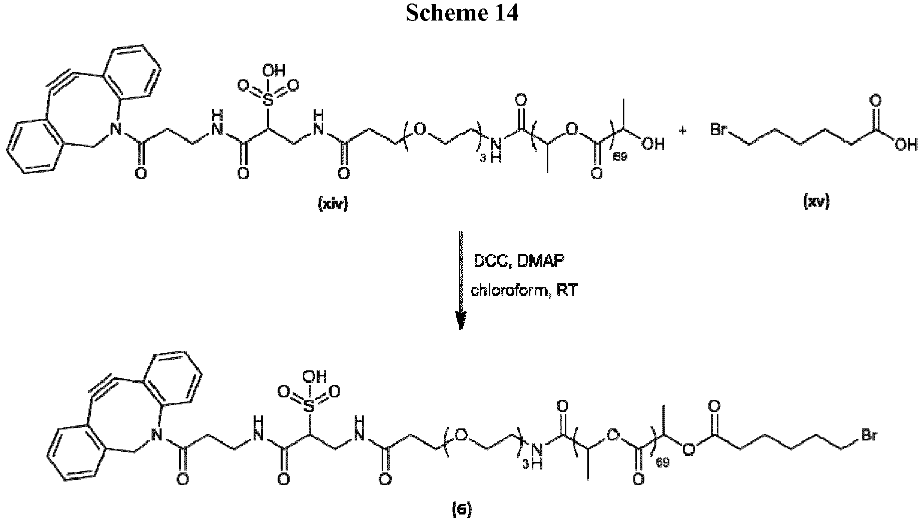

A solution of sulfo-DBCO-PEG176-PLA intermediate **(xiv)** obtained above (30 mg, 1 eq), bromohexanoic acid **(xv)** (1.2 mg, 1 eq) as functionalizable carboxylic acid (F₂-R₉-COOH **(intermediate 2)** wherein F₂ is a bromo and R₉ is pentyl), *N*,*N*'-dicyclohexylcarbodiimide (DCC) (1.4 mg, 1.1 eq) and 4-dimethylaminopyridine (DMAP) (10 mol% relative to sulfo-DBCO-PEG176-PLA intermediate) in CHCl₃ (4 mL) were reacted as described above for polymer **(1)** to give as bi-functionalizable amphiphilic di-block co-polymer of the invention, Br-PLA-PEG176-sulfo-DBCO polymer **(6).**

### Preparation of DBCO-PLGA-PEG2,000-biotin polymer (7)

A bi-functionalizable amphiphilic diblock copolymer of the invention, wherein the hydrophilic polymer is a PEG2,000 (A), the biodegradable hydrophobic polyester, a PLGA (B) and the functionalizable groups DBCO (F2) and biotin (F1) was prepared according to Scheme 1, as described in Schemes 15 and 16 below. A solution of biotin-PEG2,000-OH as starting functionalizable alcohol F₁-R₁-R₂-R₃-**A**-R₄-LH, wherein F₁ is biotin, R₂ is an amide group, A is a PEG2,000, L is O and the spacing groups R₁, R₃ and R₄ are absent) (79 mg, 1 eq) **(i)** as initiator, D,L-lactide (100 mg, 18 eq) **(ii),** glycolide (81 mg, 18 eq) **(iia)** as precursors of the hydrophilic ester polymer (PLGA), and 1,8-diazabicyclo(5.4.0)undec-7-ene (DBU) as a catalyst (2.5 mol% relative to D,L-lactide + glycolide) in CHCl₃ (0.7 mL) was irradiated in a microwave reactor and reacted as described above for intermediate **(iii)** to give the **intermediate 1b,** PLGA-PEG2,000-biotin intermediate **(xvi).**

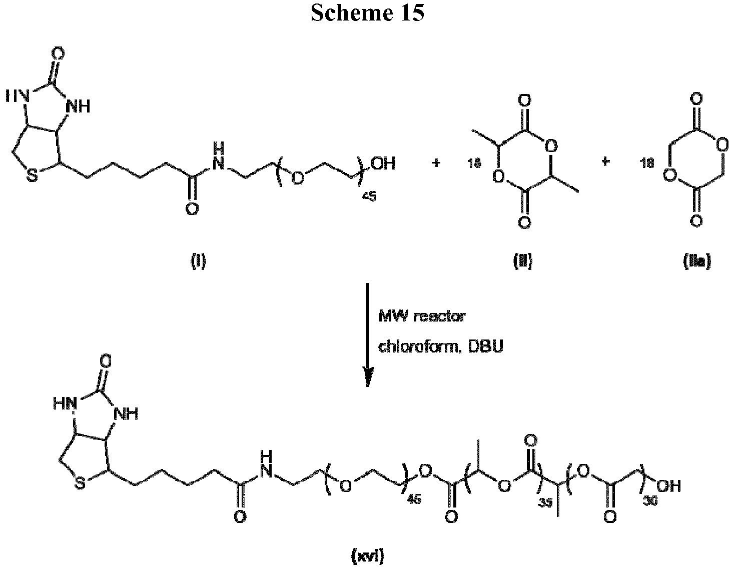

A solution of the PLGA-PEG2,000-biotin intermediate **(xv)** obtained above (75 mg, 1 eq), DBCO-COOH (3.3 mg, 1 eq) **(iv)** as functionalizable carboxylic acid (F₂-R₉-COOH **(intermediate 3)** wherein F₂ is a C₈ heterocycloalkyne such as DBCO, R₉ is ethyl carbonyl, N,N'-dicyclohexylcarbodiimide (DCC) (2.4 mg, 1.1 eq) and 4-dimethylaminopyridine (DMAP) (10 mol% relative to the PLGA-PEG2,000-biotin intermediate) in CHCl₃ (2 mL) were reacted as described above for polymer **(1)** to give as bi-functionalizable amphiphilic di-block co-polymer of the invention, the DBCO-PLGA-PEG2,000-biotin polymer **(7).**

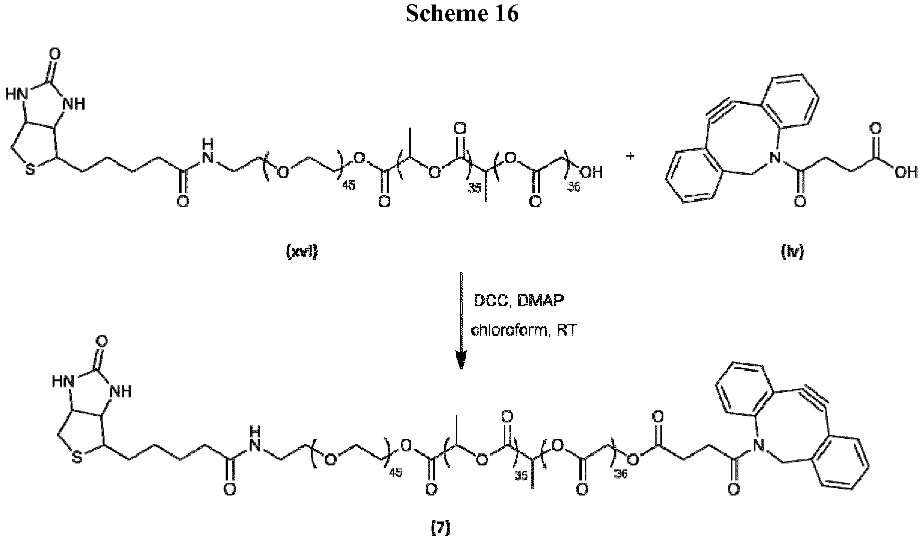

### Preparation of DBCO-PCL-PEG2,000-biotin polymer (8)

A bi-functionalizable amphiphilic diblock copolymer of the invention, wherein the hydrophilic polymer is a PEG2,000 (A), the biodegradable hydrophobic polyester, a PCL (B) and the functionalizable groups DBCO (F2) and biotin (F1) was prepared according to Scheme 1, as described in Schemes 17 and 18 below:

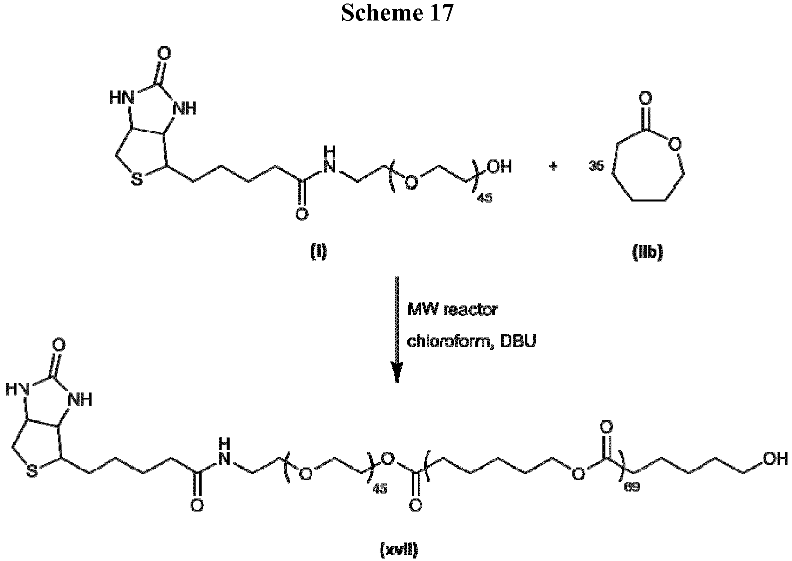

A solution of biotin-PEG2,000-OH (79 mg, 1 eq) **(i)** as starting functionalizable alcohol (F₁-R₁-R₂-R₃-**A**-R₄-LH, wherein F₁ is biotin, R₂ is an amide group, A is a PEG2,000, L is O, and the spacing groups R₁, R₃, and R₄ are absent) as initiator, caprolactone **(iib)** (158 mg, 35 eq) as precursor of the hydrophilic ester polymer (PCL) and 1,8-diazabicyclo(5.4.0)undec-7-ene (DBU) as a catalyst (2.5 mol% relative to caprolactone) in CHCl₃ (0.7 mL) was irradiated in a microwave reactor as described above for intermediate **(iii)** to give an **intermediate 1c,** PCL-PEG2,000-biotin intermediate **(xvi).**

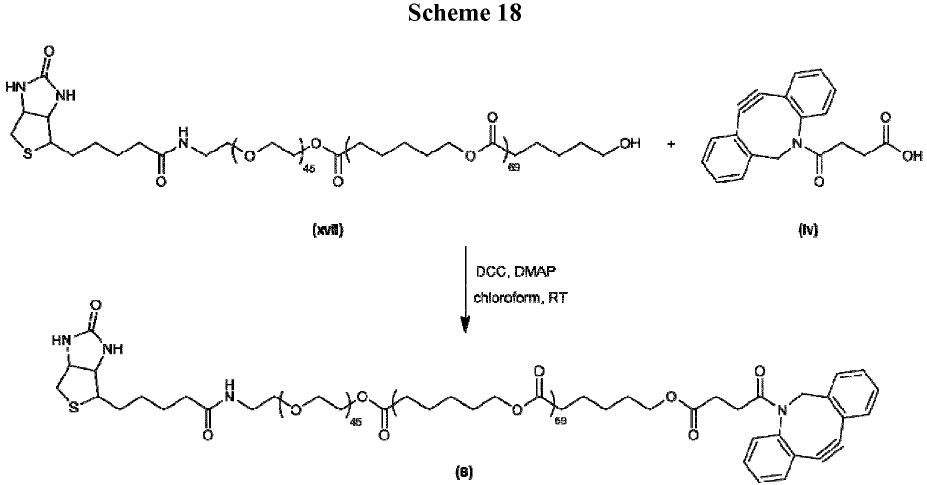

A solution of the PCL-PEG2,000-biotin intermediate **(xvi)** obtained above (75 mg, 1 eq), DBCO-COOH (3.3 mg, 1 eq) **(iv)** as functionalizable carboxylic acid (F₂-R₉-COOH **(intermediate 3)** wherein F₂ is a C₈ heterocycloalkyne such as DBCO, R₉ is ethylcarbonyl), *N*,*N*'-dicyclohexylcarbodiimide (DCC) (2.4 mg, 1.1 eq) and 4-dimethylaminopyridine (DMAP) (10 mol% relative to the PLGA-PEG2,000-biotin intermediate) in CHCl₃ (2 mL) were reacted as described above for polymer **(1)** to give as bi-functionalizable amphiphilic di-block co-polymer of the invention, the DBCO-PCL-PEG2,000-biotin polymer **(8).**

Similarly, a bi-functionalizable amphiphilic diblock copolymer of the invention can be obtained according to Scheme 2 when the biodegradable hydrophobic polyester is PLGA or PCL, similarly as for Example 4 with for example an initiation with bromoethanol.

### Preparation of DBCO-PLA-PEG2,000-biotin polymer (9)

A bi-functionalizable amphiphilic diblock copolymer of the invention, wherein the hydrophilic polymer is a PEG2,000 (A), the biodegradable hydrophobic polyester, a PLA (B) and the functionalizable groups DBCO (F2) and biotin (F1) was prepared according to Scheme 3, as described in Schemes 19 and 20 below.

A solution of poly(L-lactide) *N*-2-hydroxyethylmaleimide (200 mg, 1 eq) **(xviii)** (M₂-R₆-**B-**OH, wherein M₂ is maleimide, R₆ is hydroyethyl, B is PLA) and bromohexanol **(viii)** (Br-R₇-M₄, wherein R₇ is hexyl and M₄ is OH) (15 mg, 2 eq) are reacted with additional protection and deprotection steps of the alcohol function of bromohexanol to give the starting compound M₂-R₆-**B**-R₇-M₄ **(xix)** wherein M₂ is maleimide, R₆ is hydroyethyl, B is PLA, R₇ is hexyl and M₄ is OH.

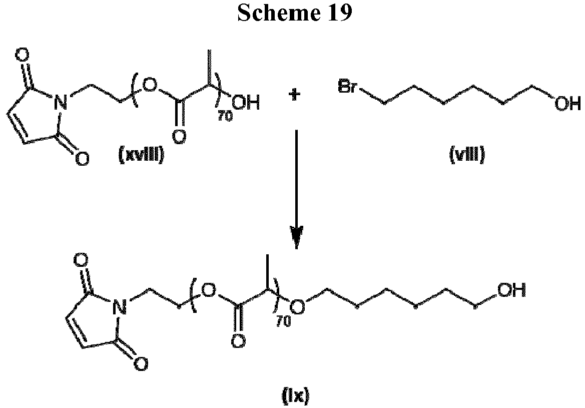

A solution of biotin-PEG2,000-SH (40 mg, 1 eq) **(xx)** (starting from F₁-R₁-R₂-R₃-**A**-R₄-M₁, wherein F₁ is biotin, R₂ is an amide group, A is a PEG2,000, M₁ is SH, and the spacing groups R₁, R₃, and R₄ are absent), intermediate **(xix)** obtained above (100 mg, 1 eq), and NEt₃ (0.1 eq) in CHCl₃ is stirred at ambient temperature for 2 h. The resulting mixture is poured in an aqueous solution of HCl 0.1 M and extracted with CH₂Cl₂, washed with brine and water.

Hexane is then added to the organic layer, and the precipitate is centrifuged for 5 min at 3,000 x *g*. After 3 cycles of dissolution of the pellet in a mixture of CH₂Cl₂/MeOH (8:2), precipitation in hexane and centrifugation for 5 min at 3,000 x *g*, the resulting pellet is precipitated in hexane and dried under reduced pressure to give the PLA-PEG2,000-biotin intermediate **(xxi) (intermediate 3a** F₁-R₁-R₂-R₃-**PEG**-R₄-R₅-R₆-**PLA**-R₇-M₄, wherein F₁ is biotin, R₂ is an amide group, R₅ is the result of the reaction between thiol and maleimide, R₆ is hydroyethyl, R₇ is hexyl and M₄ is OH, and the spacing groups R₁, R₃, and R₄ are absent).

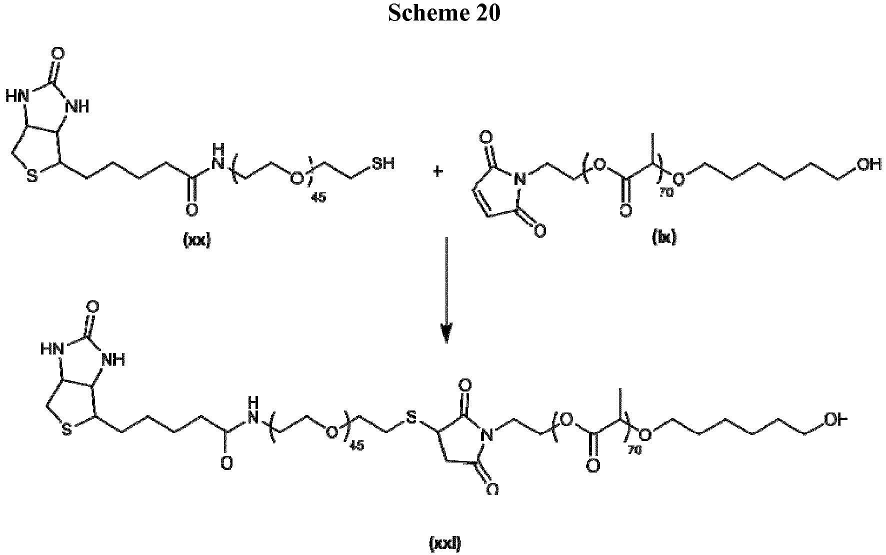

A solution of PLA-PEG2,000-biotin intermediate **(xxi)** obtained above (50 mg, 1 eq), a functionalizable carboxylic acid, DBCO-COOH **(iv)** (1 mg, 1 eq), N,N'-dicyclohexylcarbodiimide (DCC) (0.8 mg, 1.1 eq) and 4-dimethylaminopyridine (DMAP) (10 mol% relative to PLA-PEG2,000-biotin intermediate) in CHCl₃ (1 mL) are reacted as described above for polymer (1) to give as bi-functionalizable amphiphilic diblock copolymer of the invention, the DBCO-PLA-PEG2,000-biotin polymer **(9).**

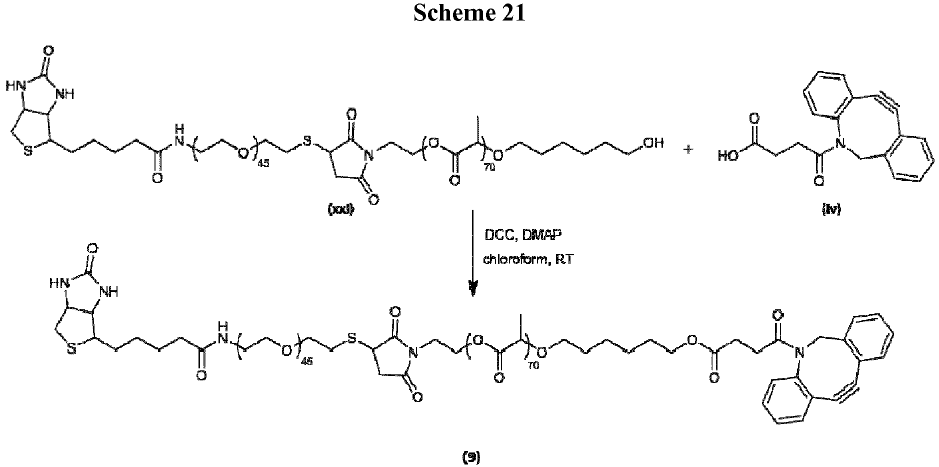

### Example 2: Characterization of polymers

The chemical structure and molecular composition of polymers were determined by proton nuclear magnetic resonance (¹H NMR) spectroscopy in deuterated chloroform (CDCl₃). The NMR experiments were carried out on a Bruker Avance III HD 600 MHz NMR spectrometer equipped with a QCI 5 mm Cryoprobe™ and a SampleJet™ automated sample changer (Bruker BioSpin, Rheinstetten, Germany). The ¹H NMR spectrum of polymer (2) described in Example 1 is presented in Figure 1 and data for the other polymers of the invention are summarized in Table 1 below.

**Table 1**

| **Polymer** | **PEG M_{w} (Da)** | **PLA M_{w} (Da)** | **Theoretical M_{w} (Da)** | **GPC M_{w} (Da)** | **NMR M_{w} (Da)** |
|---|---|---|---|---|---|
| 1 | 2,000 | 5,000 | 7,000 | 18,052 | 8,840 |
| 2 | 2,000 | 5,000 | 7,000 | 10,575 | 8,552 |
| 3 | 5,000 | 10,000 | 15,000 | 19,607 | 12,776 |
| 4 | 2,000 | 5,000 | 7,000 | 72,684 | 4,016 |

Further, the molecular weight of polymers was determined by gel permeation chromatography (GPC) after synthesis. Samples of 10 mg of polymers were dissolved in dry THF and then centrifuged for 5 min at 10,000 x g. Measurements were performed using Waters 515 HPLC pump and a Waters HPLC 717 plus autosampler coupled to a series of Styragel 7.8 x 300 mm columns (HR1 to 4 THF, Waters, Ireland). Samples were monitored using a refractive index detector (Waters 2414, Waters) with an excitation wavelength of 410 nm. The calibration curve was constructed on Waters® Millennium®32 Software, using polystyrene PSS standards polymer kit (Polymer Standards Services GmbH, Mainz, Germany). The mobile phase was THF, flowing at a rate of 1 mL·min⁻¹. The measurements were carried out in triplicates. These data support that the obtained bi-functionalizable amphiphilic di-block co-polymer of the invention have the desired structure, the expected PLA/PEG ratio and molecular weights close to the theoretical molecular weights.

### Example 3: Preparation of self-assemblies with bi-functionalizable amphiphilic polymers of the invention

Solutions of the bi-functionalizable amphiphilic di-block co-polymer of the invention (PLA-PEG **(1)** to **(4)**) in ultra-pure water were first sonicated in an ultrasonic bath sonicator TPC 120 for 1 h at 300 W (Telsonic Ultrasonics, Bronschhofen, Switzerland) and then ultra-sonicated with a Branson Digital Sonifier (Branson Ultrasonics, Danbury, USA) for 10 s and with an amplitude of 10 %. The mixture was finally centrifuged 5 min at 10,000 × g to isolate the supernatant containing the self-assemblies. The self-assemblies size was determined by dynamic light scattering measurements carried out with a Zetasizer 3000 (Malvern Instruments, Ltd., U.K.) in ultra-pure water over 12 runs of 10 s each for size carried out in triplicates.

**Table 2**

| **Polymer** | **Self-assemblies mean size (nm)** | **SD** |
|---|---|---|
| 1 | 251.7 | 8.1 |
| 2 | 178.0 | 3.0 |
| 3 | 189.3 | 2.5 |
| 4 | 131.0 | 2.6 |

Those data support that the obtained bi-functionalizable amphiphilic di-block co-polymers of the invention can easily form nano self-assemblies.

### Example 4: Preparation of conjugates with bi-functionalizable amphiphilic polymers of the invention

Conjugates of the invention were prepared starting with a bi-functionalizable amphiphilic di-block co-polymer of the invention (2) and the following fluorophores as bioactive agents: streptavidin-*Alexa Fluor*™ 488, FITC-thiol, cyanine7-DBCO and cyanine7-azide. Streptavidin-*Alexa Fluor*™ 488 was purchased from Thermo Fisher Scientific, FITC-thiol was purchased from Nanocs Inc., cyanine7-DBCO was purchased from Jena Bioscience, and cyanine7-azide was purchased from Lumiprobe.

### a) Conjugation of Alexa F/uor™ 488 (F₁ side) and cyanine7 (F₂ side) on a bi-functionalizable amphiphilic di-block co-polymer of the invention

Solutions of streptavidin-*Alexa Fluor*™ 488 and cyanine7-DBCO in ultra-pure water were added to a solution of N₃-PLA-PEG-biotin polymer **(2)** self-assemblies in ultra-pure water. The resulting mixture was ultra-sonicated with a Branson Digital Sonifier (Branson Ultrasonics, Danbury, USA) for 30 s and with an amplitude of 10 % and then sonicated in an ultrasonic bath sonicator TPC 120 for 1 h at 300 W (Telsonic Ultrasonics, Bronschhofen, Switzerland). Purification by centrifugation (5 min, 3,000 x *g*) (centrifuge PK121, ALC, Cologno Monzese, Italy) using Spin-X UF 6 (Corning, Corning, USA) with an MWCO of ca. 100,000 Da was finally necessary to obtain solution of bi-functionalized PLA-PEG polymers **(C1)** self-assemblies (conjugate of the invention wherein the hydrophilic polymer is a PEG2,000 (A), the biodegradable hydrophobic polyester is PLA (B), R₂ is an amide group, R₅ is an ester, R₈ is an ester R₉ is methyl and R₁, R₃, R₄, R₆, and R₇ are absent and the functional groups F₃ and F₄ being the result of the coupling of F₁ and F₂ with streptavidin-*Alexa Fluor*™ 488 and Cyanine 7-DBCO, respectively).

### b) Conjugation of FITC (F₁ side) and cyanine7 (F₂ side) on a bi-functionalizable amphiphilic di-block co-polymer of the invention

Solutions of FITC-thiol and cyanine7-azide in ultra-pure water were added to a solution of DBCO-PLA-PEG5,000-maleimide polymer self-assemblies in ultra-pure water. The resulting mixture was ultra-sonicated with a Branson Digital Sonifier (Branson Ultrasonics, Danbury, USA) for 30 s and with an amplitude of 10 % and then sonicated in a bath sonicator for 1 h at 300 W (Telsonic Ultrasonics, Bronschhofen, Switzerland). Purification by dialysis against ultra-pure water using tubing with an MWCO of ca. 3,500 Da was finally necessary to obtain solution of bi-functionalized PLA-PEG polymers **(C2)** self-assemblies (conjugate of the invention wherein the hydrophilic polymer is a PEG2,000 (A), the biodegradable hydrophobic polyester is PLA (B), R₁ is an ethyl, R₂ is an amide, R₅ is an ester, R₈ is an ester R₉ is ethyl carbonyl and R₃, R₄, R₆ and R₇ are absent and the functional groups F₃ and F₄ being the result of the coupling of F₁ and F₂ with FITC-thiol and Cyanine 7-azide, respectively).

### c) Characterization of the self-assemblies made of bi-functionalized amphiphilic polymers

Fluorescence of self-assemblies solution of bi-functionalized PLA-PEG polymers obtained above was measured before and after dialysis in a plate reader (Synergy Mx with software Gen5 2.00, BioTek, Winooski, VT, USA) in order to estimate the amount of fluorophores linked to the polymers. The measurement was carried out on 100 µL samples from the bottom with an excitation wavelength of 490 nm (9.0), an emission wavelength of 525 nm (9.0) and a gain of 80 to detect Alexa Fluor™ 488-functionalized polymers and FITC-functionalized polymers and with an excitation wavelength of 750 nm (9.0), an emission wavelength of 773 nm (9.0) and a gain of 80 to detect cyanine7-functionalized polymers. This experiment was carried out in triplicates.

**Table 3**

| **Example** | **Fluorophores** | **Excitation /emission wavelength** | **Remaining fluorophores after dialysis (%)** |
|---|---|---|---|
| C1 | Streptavidin-Alexa Fluor™ 488 + cyanine7-DBCO | 490/525 | 64.2 |
| C2 | FITC-thiol + cyanine7-azide | | 72.8 |
| C1 | Streptavidin-Alexa Fluor™ 488 + cyanine7-DBCO | 750/773 | 70.1 |
| C2 | FITC-thiol + cyanine7-azide | | 62.4 |

These data support that it is possible to prepare conjugates of the invention already in a self-assembly state in an aqueous medium. In this example, the bioactive agent is a fluorophore. The fluorophores both outside and within the self-assemblies were detected by a plate reader which is a clear advantage for following up of the fate of the nano/micro-carriers during experiments.

## Claims

1. A bi-functionalizable amphiphilic di-block co-polymer comprising:
- a hydrophilic polymer;
- a biodegradable hydrophobic polyester; and
- two functionalizable groups at each end of the said amphiphilic di-block co-polymer, wherein said functionalizable groups can be **(i)** conjugated by addition reaction, in particular, a click reaction, in aqueous media to another reacting moiety suitable for click chemistry or **(ii)** coupled to a reacting group having a specific affinity for said functionalizable group form a complex; or any pharmaceutically acceptable salts thereof.

2. A bi-functionalizable according to claim 1, of Formula (I):
**F₁**-R₁-R₂-R₃-**A**-R₄-R₅-R₆-**B**-R₇-R₈-R₉-**F₂₁** **(I)**
wherein F₁ and F₂ are two functionalizable groups which can be conjugated by click reaction in aqueous media to another reacting moiety suitable for click chemistry; R₂, R₅, and R₈, are linking groups; R₁, R₃, R₄, R₆, R₇, and R₉ are optional spacing groups; **A** is a hydrophilic polymer and **B** is a biodegradable hydrophobic polyester or any pharmaceutically acceptable salts thereof.

3. A bi-functionalizable according to any one of claims 1 to 2, wherein the said biodegradable hydrophobic polyester is selected from polylactic acid (PLA), poly lactic-co-glycolic acid (PLGA) and poly-ε-caprolactone (PCL) or a derivative thereof.

4. A bi-functionalizable according to any one of claims 1 to 3, wherein F₁ and F₂ are two functionalizable groups independently selected from the following group: C₈ cycloalkynyls, Cg heterocycloalkynyls C₂-C₆ alkynes, azides, carboxylic acids, thiols, maleimide, biotin, avidin, halogen, amines, acrylates, active esters, epoxides, folic acid, hydrazides, nitrophenyl carbonate, ortho-pyridyl disulfide, protected amines (Fmoc, tBOC), vinylsulfone, sulfonate (tosyl, mesyl) and silanes.

5. A bi-functionalizable according to any one of claims 1 to 4, wherein the said hydrophilic polymer is a PEG or a derivative thereof.

6. A bi-functionalizable according to any one of claims 2 to 5, wherein the optional spacing groups R₁, R₃, R₄, R₆, R₇, and R₉, when present, are independently selected from optionally substituted C₁-C₆ alkyl, optionally substituted C₂-C₈ alkenyl or optionally substituted C₂-C₈ alkynyl groups, optionally interrupted by at least one heteroatom selected among Si, O, N and S; optionally substituted aryl groups; optionally substituted amino acid; optionally substituted optionally subsituted peptidic sequence; optionally substituted C₁-C₆ alkoxy groups; and optionally substituted amino groups, wherein the optional substituting groups are selected from the following list: halogens; hydroxylamine (-ONH₂) group, hydrazine (-NH-NH₂) group; nitro (-NO₂); azido (-N₃) group, diazonium (-N₂⁺) group, optionally in presence of a counterion, alkyl- or optionally substituted alkoxycarbonyl, optionally subsituted acyl; hydroxyl (-OH) group, boronic acid -B(OR")₂ group; wherein R" is a hydrogen atom or an alkyl group; phosphine or phosphonium groups; isocyanate (-N=C=O) or isothiocyanate (-N=C=S) group; chlorosulfonyl (-SO₂Cl) group; O-C(=O)-C(N₂)-CF₃ group or a -C(=O)-C(N₂)-CF₃ group; activated esters, such as -C(=O)-NHS, wherein NHS stands for N-hydrosuccinimidyl; perfluorinated esters and acylureas and-a C=C-C=N group.

7. A bi-functionalizable according to any one of claims 2 to 6, wherein the linking groups R₂, R₅, and R₈ are independently a product of the reaction of two groups from the following list consisting in:
acetal, acid anhydride, acyl, acylal, acyloin, acylsilane, aldimine, alkoxy, amides, amines, amine oxide, azo compound, azoxy, bisthiosemicarbazone, borinic acid, boronic ester, borinic ester, carbodiimide, carbonate ester, carbonyl, carboxamide, cumulene, cyanohydrins, disulfide, enamines, enol ethers, enolate anion, enone, enyne, episulfide, epoxy, ester, ether, glycoside bonds, hemiacetal, hemiaminal, hemiketal, hydrazone, hydroxamic acid, heterocycle, imine, imide, iminium, ketal, ketone, methine, methylenedioxy, n-oxoammonium, nitroamine, nitronate, nitrone, nitrosamine, organic peroxide, orthoester, oxime, phenylene, phosphinate, phosphine oxide, phosphine, phosphodiester, phosphinite, phosphite, phosphonate, phosphonite, phosphorane, semicarbazone, silyl enol ethers, silyl ethers, sulfide, sulfonamide, sulfone, sulfoxide, thioacetal, thioamide, thioester, thioether, thioketal, thioketone, thiourea, triazene, urea, xanthate.

8. A bi-functionalizable according to any one of claims 1 to 7, wherein the functionalizable groups are selected from biotin, alkynes such as C₈ cycloalkynyl or Cg heterocycloalkynyl (e.g., DBCO), azides such as azidoacetic acid, maleimide, and halide.

9. A bi-functionalizable amphiphilic di-block co-polymer according to any one of claims 1 to 8, selected from the following group:
a DBCO-PLA-PEG2,000-biotin polymer;
a N3-PLA-PEG2,000-biotin polymer;
a DBCO-PLA-PEG5,000-maleimide polymer;
a Br-PLA-PEG2,000-maleimide polymer;
a DBCO-PLA-PEG2,000-maleimide polymer;
a Br-PLA-PEG176-sulfo-DBCO polymer;
a DBCO-PLGA-PEG2,000-biotin polymer; and
a DBCO-PCL-PEG2,000-biotin polymer or any pharmaceutically acceptable salts thereof.

10. A bi-functionalizable amphiphilic di-block co-polymer to any one of claims 1 to 9 having an average molecular weight from about 300 to 1,000,000 Da and about 8,000 to 80,000 Da.

11. A conjugate comprising a bi-functionalizable amphiphilic di-block copolymer according to any one of claims 1 to 9 conjugated with at least one bioactive active agent.

12. A conjugate of claim 1, according to Formula (II):
**F**₃-R₁-R₂-R₃-**A**-R₄-R₅-R₆-**B**-R₇-R₈-R₉-**F**₄ **(II)**
wherein F₃ and F₄ are moieties resulting from the coupling of a bioactive agent with a functionalizable group F₁ and F₂, respectively, through an addition reaction (e.g., "click") or from the interaction of a functionalizable group F₁ and F₂ with a reacting group of a bioactive agent, said reacting group having a specific affinity for said functionalizable group F₁ and F₂; F₁, F₂, R₁, R₂, R₃, R₄, R₅, R₆, R₇, R₈, and R₉; **A** and **B** are as described as defined any of the above claims or any pharmaceutically acceptable salts thereof.

13. A pharmaceutical or cosmetic composition comprising at least one bi-functionalizable amphiphilic di-block copolymer according to any one of claims 1 to 9 or at least one conjugate according to claim 10 or 11 and at least one pharmaceutically acceptable carrier, diluent or excipient thereof.

14. A pharmaceutical or cosmetic composition according to claim 12 or a conjugate of according to any one of claims 10 to 11 for use in *in vivo* delivery of medical agents, the diagnosis or prevention and/or treatment of disease or medical disorder, *in vitro* cell culture and tissue engineering applications.

15. A process for the preparation of a bi-functionalizable amphiphilic di-block copolymer according to any one of claims 1 to 9, said process comprising:
(i) the steps of:
(a) Providing a precursor of a biodegradable hydrophobic polyester **B** (e.g., lactide, lactide, and glycolide or caprolactone);
(b) Reacting the said precursor with a compound F₁-R₁-R₂-R₃-**A**-R₄-LH to lead to an intermediate monofunctionalizable amphiphilic di-block co-polymer F₁-R₁-R₂-R₃-**A**-R₄-R₅-R₆-**B**-R₇-OH **(intermediate 1**);
(c) Reacting the said intermediate 1 with a functionalizable carboxylic acid of formula F₂-R₉-COOH **(intermediate 3**) under esterification condition to lead to a bi-functionalizable amphiphilic di-block co-polymer according to any one of claims 1 to 9, wherein F₁, F₂, R₁, R₂, R₃, R₄, R₅, R₆, R₇, R₈, and R₉; **A** and **B** are as described as defined any of the above claims;
or
(ii) the steps of:
a) Providing a precursor of a biodegradable hydrophobic polyester **B** (e.g., lactide, lactide, and glycolide or caprolactone);
b) Reacting the said precursor with a functionalizable alcohol of formula F₂-R₉-LH to lead to an intermediate monofunctionalizable hydrophobic polymer intermediate co-polymer F₂-R₉-R₈-R₇-**B**-R₆-OH **(intermediate 2**);
c) Reacting the said intermediate 2 with a functionalizable hydrophilic polymer F₁-R₁-R₂-R₃-**A**-R₄-COOH under esterification condition to lead to a bi-functionalizable amphiphilic di-block co-polymer according to any one of claims 1 to 9, wherein F₁, F₂, R₁, R₂, R₃, R₄, R₅, R₆, R₇, R₈, and R₉; **A** and **B** are as described as defined any of the above claims.

16. A kit for the preparation of nanocarriers for encapsulation of material, said kit comprising:
i) a precursor of a biodegradable hydrophobic polyester **B** (e.g., lactide, lactide, and glycolide or caprolactone); and
ii) precursors compounds F₁-R₁-R₂-R₃-**A**-R₄-LH and F₂-R₉-COOH;
or
iii) precursors compounds F₂-R₉-LH and functionalizable hydrophilic polymer F₁-R₁-R₂-R₃-**A**-R₄-COOH; and
iv) optionally a catalyst for ring opening reactions.
